# EUROPEAN PATENT APPLICATION

(11) **EP 4 089 073 A1**
(43) Date of publication of application: **16.11.2022**
(21) Application number: 22171897.6
(22) Date of filing: 05.05.2022
(51) Int. Cl.: C07D 209/88, C09K 11/06, H01L 51/50

(54) **LUMINESCENCE DEVICE AND NITROGEN-CONTAINING COMPOUND FOR A LUMINESCENCE DEVICE**

(30) Priority: 12.05.2021 KR 20210061617
(71) Applicant: Samsung Display Co., Ltd., Gyeonggi-Do (KR)
(72) Inventor: HAN, Sanghyun, Hwaseong-si (KR); KIM, Dongjun, Suwon-si (KR); KIM, Minji, Hwaseong-si (KR); PAK, Hankyu, Suwon-si (KR); JEONG, Eunjae, Hwaseong-si (KR); JO, Sohee, Cheonan-si (KR)
(74) Representative: Marks & Clerk LLP

(57) **Abstract**

A luminescence device includes a first electrode, a hole transport region disposed on the first electrode, an emission layer disposed on the hole transport region, an electron transport region disposed on the emission layer, and a second electrode disposed on the electron transport region. The hole transport region contains a nitrogen-containing compound represented by Formula 1, and thus, the luminescence device may exhibit long lifespan and high efficiency. The substituents are the same as described in the detailed descriptions.

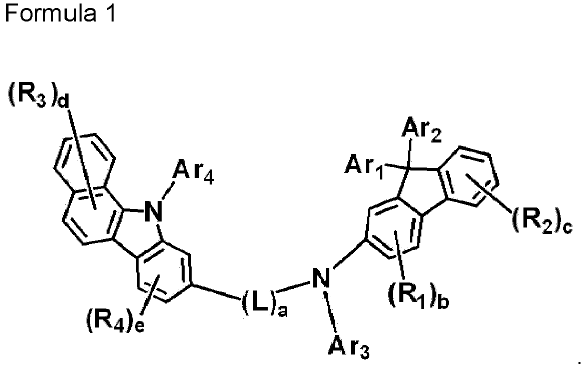

## Description

### BACKGROUND

The present disclosure herein relates to a luminescence device and a nitrogen-containing compound for a luminescence device.

As image display apparatuses, luminescence displays have been actively developed lately. Unlike liquid crystal displays, the luminescence displays are so-called self-luminescent display apparatuses in which holes and electrons injected from a first electrode and a second electrode recombine in an emission layer, and thus, a luminescent material in the emission layer emits light to accomplish display (e.g., to display an image).

For application of luminescence devices in display apparatuses, there is a demand for luminescence devices having a low driving voltage, high luminous efficiency, and long lifespan, and development of materials for luminescence devices capable of stably attaining such characteristics is being continuously pursued.

### SUMMARY

Aspects according to one or more embodiments of the present disclosure are directed toward a luminescence device and a nitrogen-containing compound for a luminescence device, and more particularly, a highly efficient luminescence device and a nitrogen-containing compound included in a hole transport region of the luminescence device.

According to an embodiment of the present disclosure, a nitrogen-containing compound is represented by Formula 1 below.

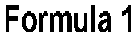

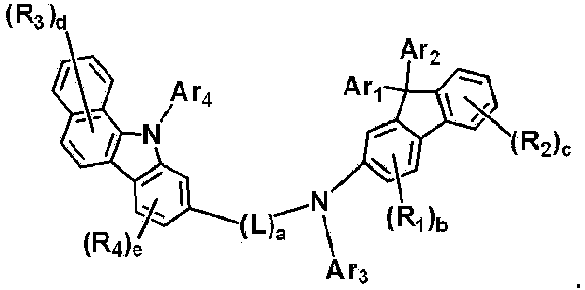

In Formula 1 above, L is a direct linkage, a substituted or unsubstituted divalent hydrocarbon ring group having 6 to 30 ring-forming carbon atoms, a substituted or unsubstituted arylene group having 6 to 30 ring-forming carbon atoms, a substituted or unsubstituted divalent aliphatic heterocyclic group having 2 to 30 ring-forming carbon atoms, or a substituted or unsubstituted heteroarylene group having 2 to 30 ring-forming carbon atoms; Ar₁ to Ar₄ are each independently a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; R₁ to R₄ are each independently a hydrogen atom, a deuterium atom, a halogen atom, a nitro group, a cyano group, an oxy group, a substituted or unsubstituted amine group, a substituted or unsubstituted thio group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; a, b, and e are each independently an integer of 0 to 3; c is an integer of 0 to 4; and d is an integer of 0 to 6.

Ar₁ to Ar₄ above may be each independently a substituted or unsubstituted aryl group having 6 to 30 ring-forming carbon atoms.

L in Formula 1 may be a direct linkage or a substituted or unsubstituted arylene group having 6 to 18 ring-forming carbon atoms.

The nitrogen-containing compound represented by Formula 1 above may be represented by Formula 2-1 or Formula 2-2 below.

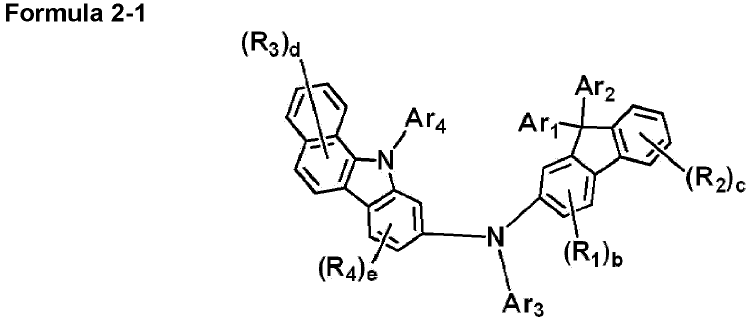

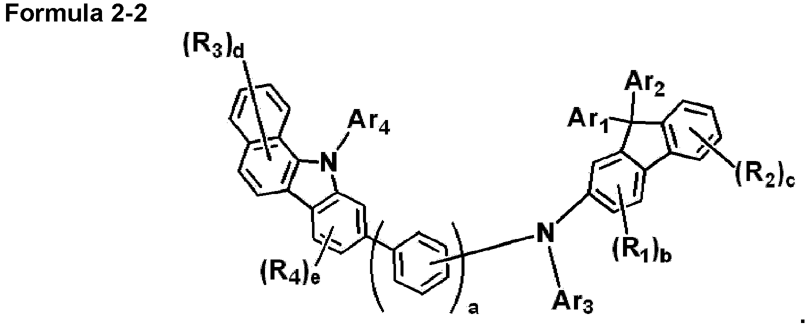

In Formulae 2-1 and 2-2, Ar₁ to Ar₄, R₁ to R₄, and a to e are the same as respectively defined in Formula 1.

The nitrogen-containing compound represented by Formula 1 above may be represented by Formula 3 below.

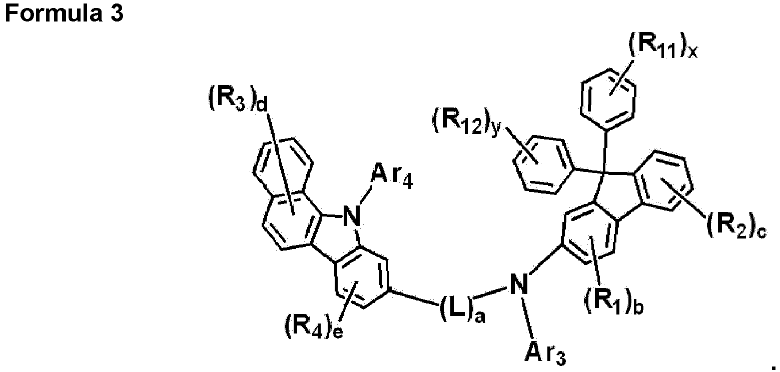

In Formula 3 above, R₁₁ and R₁₂ are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring, x and y are each independently an integer of 0 to 5, and Ar₃, Ar₄, R₁ to R₄, L, and a to e are the same as respectively defined in Formula 1.

The nitrogen-containing compound may be a monoamine compound.

Ar₃ and Ar₄ above may be each independently represented by any one of Formulae 4-1 to 4-4 below.

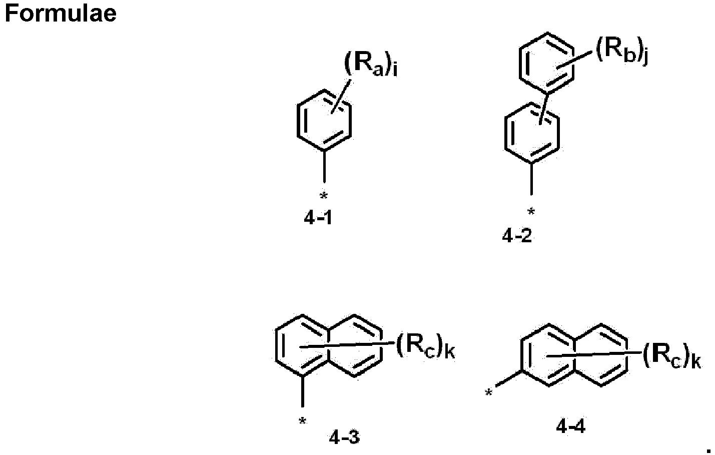

In Formulae 4-1 to 4-4 above, Rₐ to R_{c} are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring, i and j are each independently an integer of 0 to 5, and k is an integer of 0 to 7.

The nitrogen-containing compound represented by Formula 3 above may be represented by Formula 5 below.

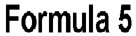

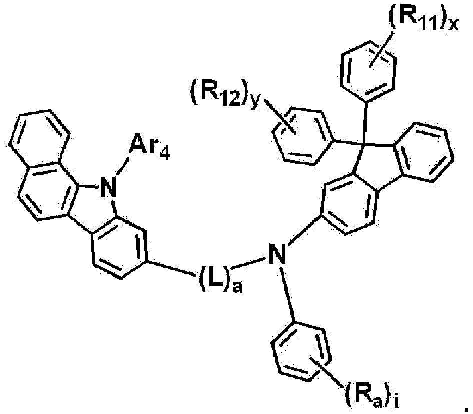

In Formula 5 above, Rₐ is a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring, i is an integer of 0 to 5, and Ar₄, R₁₁, R₁₂, L, a, x, and y are the same as respectively defined in Formula 3.

The nitrogen-containing compound represented by Formula 1 above may be at least one selected from compounds represented by Compound Group 1.

According to an embodiment of the present disclosure, a luminescence device includes a first electrode, a hole transport region on the first electrode, an emission layer on the hole transport region, an electron transport region on the emission layer, and a second electrode on the electron transport region, wherein the hole transport region includes the nitrogen-containing compound.

In an embodiment, the hole transport region may include a hole injection layer on the first electrode and a hole transport layer on the hole injection layer, wherein at least one of the hole injection layer or the hole transport layer may include the nitrogen-containing compound.

In an embodiment, the hole transport region may include a hole transport layer on the first electrode and an electron blocking layer on the hole transport layer, wherein the electron blocking layer may contain the nitrogen-containing compound of an embodiment.

In an embodiment, the emission layer may include a polycyclic compound represented by Formula E-1 below.

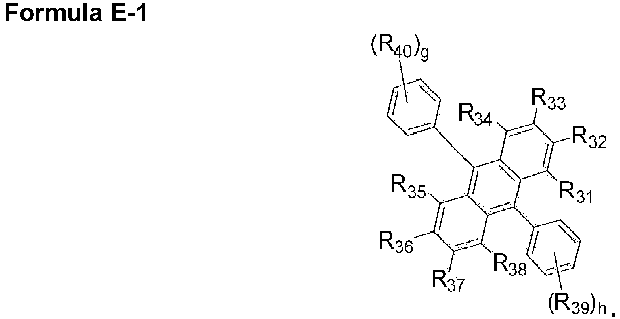

In Formula E-1 above, R₃₁ to R₄₀ are each independently a hydrogen atom, a deuterium atom, a halogen atom, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring, and g and h are each independently an integer of 0 to 5.

At least some of the above and other features of the invention are set out in the claims.

### BRIEF DESCRIPTION OF THE FIGURES

The accompanying drawings are included to provide a further understanding of the present disclosure, and are incorporated in and constitute a part of this specification. The drawings illustrate embodiments of the present disclosure and, together with the description, serve to explain principles of the present disclosure. In the drawings:
FIG. 1 is a plan view of a display apparatus according to an embodiment of the present disclosure;
FIG. 2 is a cross-sectional view of a display apparatus according to an embodiment of the present disclosure;
FIG. 3 is a cross-sectional view schematically showing a luminescence device according to an embodiment of the present disclosure;
FIG. 4 is a cross-sectional view schematically showing a luminescence device according to an embodiment of the present disclosure;
FIG. 5 is a cross-sectional view schematically showing a luminescence device according to an embodiment of the present disclosure;
FIG. 6 is a cross-sectional view schematically showing a luminescence device according to an embodiment of the present disclosure;
FIG. 7 is a cross-sectional view of a display apparatus according to an embodiment of the present disclosure; and
FIG. 8 is a cross-sectional view of a display apparatus according to an embodiment of the present disclosure.

### DETAILED DESCRIPTION

The present disclosure may be modified in many alternate forms, and thus specific embodiments will be illustrated in the drawings and described in more detail. It should be understood, however, that it is not intended to limit the present disclosure to the particular forms disclosed, but rather, is intended to cover all modifications, equivalents, and alternatives falling within the scope of the present disclosure.

In describing the drawings, like reference numerals are used to refer to like elements. In the drawings, the sizes of elements may be exaggerated for clarity. It will be understood that, although the terms "first", "second", etc. may be used herein to describe various elements, these elements should not be limited by these terms. These terms are only used to distinguish one element from another. For example, a first element could be termed a second element, and, similarly, a second element could be termed a first element, without departing from the scope of example embodiments of the present disclosure. The terms of a singular form may include plural forms unless the context clearly indicates otherwise.

In the present description, it should be understood that the terms "comprise", or "have" are intended to specify the presence of stated features, integers, steps, operations, elements, components, or combinations thereof in the disclosure, but do not preclude the presence or addition of one or more other features, integers, steps, operations, elements, components, or combinations thereof.

In the present description, it should be understood that when an element such as a layer, a film, a region, or a substrate is referred to as being "on" or "above" another element, it may be "directly on" the other element or intervening element(s) may also be present. Also, it should be understood that when an element such as a layer, a film, a region, or a substrate is referred to as being "beneath" or "under" another element, it may be "directly under" the other element or intervening element(s) may also be present. In addition, in the present description, it should be understood that when an element is referred to as being "on" another element, it may be "above" or "under" the other element.

In the present description, the term "substituted or unsubstituted" may indicate that a group is unsubstituted, or substituted with at least one substituent selected from the group consisting of a deuterium atom, a halogen atom, a cyano group, a nitro group, an amine group, a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, an alkoxy group, a hydrocarbon ring group, an aryl group, and a heterocyclic group. In addition, each of the substituents described above may be substituted or unsubstituted. For example, a biphenyl group may be interpreted as an aryl group or as a phenyl group substituted with a phenyl group.

In the present description, the term "bonded to an adjacent group to form a ring" may indicate that a group is bonded to an adjacent group to form a substituted or unsubstituted hydrocarbon ring, or a substituted or unsubstituted heterocycle. The hydrocarbon ring includes an aliphatic hydrocarbon ring and an aromatic hydrocarbon ring. The heterocycle includes an aliphatic heterocycle and an aromatic heterocycle. The hydrocarbon ring and the heterocycle may be monocyclic or polycyclic. In addition, the rings formed by being bonded to each other may be connected to another ring to form a spiro structure.

In the present description, the term "an adjacent group" may refer to a substituent substituted for an atom which is directly connected to an atom substituted with a corresponding substituent, another substituent substituted for an atom which is substituted with a corresponding substituent, or a substituent sterically positioned at the nearest position to a corresponding substituent. For example, two methyl groups in 1,2-dimethylbenzene may be interpreted as mutually "adjacent groups" and two ethyl groups in 1,1-diethylcyclopentane may be interpreted as mutually "adjacent groups". In addition, two methyl groups in 4,5-dimethylphenanthrene may be interpreted as mutually "adjacent groups".

In the present description, examples of a halogen atom may include a fluorine atom, a chlorine atom, a bromine atom, and an iodine atom.

In the present description, an alkyl group may be a linear, branched or cyclic alkyl group. The number of carbon atoms in the alkyl group may be 1 to 50, 1 to 30, 1 to 20, 1 to 10, or 1 to 6. Examples of the alkyl group may include a methyl group, an ethyl group, an n-propyl group, an isopropyl group, an n-butyl group, a s-butyl group, a t-butyl group, an i-butyl group, a 2-ethylbutyl group, a 3,3-dimethylbutyl group, an n-pentyl group, an i-pentyl group, a neopentyl group, a t-pentyl group, a cyclopentyl group, a 1-methylpentyl group, a 3-methylpentyl group, a 2-ethylpentyl group, a 4-methyl-2-pentyl group, an n-hexyl group, a 1-methylhexyl group, a 2-ethylhexyl group, a 2-butylhexyl group, a cyclohexyl group, a 4-methylcyclohexyl group, a 4-t-butylcyclohexyl group, an n-heptyl group, a 1-methylheptyl group, a 2,2-dimethylheptyl group, a 2-ethylheptyl group, a 2-butylheptyl group, an n-octyl group, a t-octyl group, a 2-ethyloctyl group, a 2-butyloctyl group, a 2-hexyloctyl group, a 3,7-dimethyloctyl group, a cyclooctyl group, an n-nonyl group, an n-decyl group, an adamantyl group, a 2-ethyldecyl group, a 2-butyldecyl group, a 2-hexyldecyl group, a 2-octyldecyl group, an n-undecyl group, an n-dodecyl group, a 2-ethyldodecyl group, a 2-butyldodecyl group, a 2-hexyldodecyl group, a 2-octyldodecyl group, an n-tridecyl group, an n-tetradecyl group, an n-pentadecyl group, an n-hexadecyl group, a 2-ethylhexadecyl group, a 2-butylhexadecyl group, a 2-hexylhexadecyl group, a 2-octylhexadecyl group, an n-heptadecyl group, an n-octadecyl group, an n-nonadecyl group, an n-eicosyl group, a 2-ethyleicosyl group, a 2-butyleicosyl group, a 2-hexyleicosyl group, a 2-octyleicosyl group, an n-heneicosyl group, an n-docosyl group, an n-tricosyl group, an n-tetracosyl group, an n-pentacosyl group, an n-hexacosyl group, an n-heptacosyl group, an n-octacosyl group, an n-nonacosyl group, an n-triacontyl group, etc., but the present disclosure is not limited thereto.

In the present description, a hydrocarbon ring group refers to any functional group or substituent derived from an aliphatic hydrocarbon ring. The hydrocarbon ring group may be a saturated hydrocarbon ring group having 5 to 20 ring-forming carbon atoms.

In the present description, an aryl group refers to any functional group or substituent derived from an aromatic hydrocarbon ring. The aryl group may be a monocyclic aryl group or a polycyclic aryl group. The number of ring-forming carbon atoms in the aryl group may be 6 to 36, 6 to 30, 6 to 20, or 6 to 15. Examples of the aryl group may include a phenyl group, a naphthyl group, a fluorenyl group, an anthracenyl group, a phenanthryl group, a biphenyl group, a terphenyl group, a quaterphenyl group, a quinquephenyl group, a sexiphenyl group, a triphenylenyl group, a pyrenyl group, a benzofluoranthenyl group, a chrysenyl group, etc., but the present disclosure is not limited thereto.

In the present description, a fluorenyl group may be substituted, and two substituents may be bonded to each other to form a spiro structure. An example that the fluorenyl group is substituted is as follows. However, the embodiment of the present disclosure is not limited thereto.

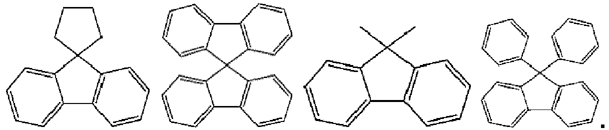

In the present description, a heterocyclic group refers to any functional group or substituent derived from a ring containing at least one of B, O, N, P, Si, or S as a hetero atom. The heterocyclic group includes an aliphatic heterocyclic group and an aromatic heterocyclic group. The aromatic heterocyclic group may be a heteroaryl group. The aliphatic heterocycle and the aromatic heterocycle may be monocyclic or polycyclic.

In the present description, the heterocyclic group may contain at least one of B, O, N, P, Si, or S as a hetero atom. When the heterocyclic group contains two or more hetero atoms, the two or more hetero atoms may be the same as or different from each other. The heterocyclic group may be a monocyclic heterocyclic group or a polycyclic heterocyclic group, and include a heteroaryl group. The number of ring-forming carbon atoms in the heterocyclic group may be 2 to 30, 2 to 20, or 2 to 10.

In the present description, the aliphatic heterocyclic group may contain at least one of B, O, N, P, Si, or S as a hetero atom. The number of ring-forming carbon atoms in the aliphatic heterocyclic group may be 2 to 30, 2 to 20, or 2 to 10. Examples of the aliphatic heterocyclic group may include an oxirane group, a thiirane group, a pyrrolidine group, a piperidine group, a tetrahydrofuran group, a tetrahydrothiophene group, a thiane group, a tetrahydropyran group, a 1,4-dioxane group, etc., but the present disclosure is not limited to thereto.

In the present description, a heteroaryl group may include at least one of B, O, N, P, Si, or S as a hetero atom. When the heteroaryl group contains two or more hetero atoms, the two or more hetero atoms may be the same as or different from each other. The heteroaryl group may be a monocyclic heteroaryl group or a polycyclic heteroaryl group. The number of ring-forming carbon atoms in the heteroaryl group may be 2 to 30, 2 to 20, or 2 to 10. Examples of the heteroaryl group may include a thiophene group, a furan group, a pyrrole group, an imidazole group, a triazole group, a pyridine group, a bipyridine group, a pyrimidine group, a triazine group, an acridyl group, a pyridazine group, a pyrazinyl group, a quinoline group, a quinazoline group, a quinoxaline group, a phenoxazine group, a phthalazine group, a pyrido pyrimidine group, a pyrido pyrazine group, a pyrazino pyrazine group, an isoquinoline group, an indole group, a carbazole group, an N-arylcarbazole group, an N-heteroarylcarbazole group, an N-alkylcarbazole group, a benzoxazole group, a benzoimidazole group, a benzothiazole group, a benzocarbazole group, a benzothiophene group, a dibenzothiophene group, a thienothiophene group, a benzofuran group, a phenanthroline group, a thiazole group, an isoxazole group, an oxazole group, an oxadiazole group, a thiadiazole group, a phenothiazine group, a dibenzosilole group, a dibenzofuran group, etc., but the present disclosure is not limited thereto.

In the present description, the above description of the aryl group may be applied to an arylene group, except that the arylene group is a divalent group. The above description of the heteroaryl group may be applied to a heteroarylene group, except that the heteroarylene group is a divalent group.

In the present description, a silyl group includes an alkyl silyl group and an aryl silyl group. Examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, etc., but the present disclosure is not limited thereto.

In the present description, a thio group may include an alkyl thio group and an aryl thio group. The thio group may refer to a group in which a sulfur atom is bonded to an alkyl group or an aryl group as defined above. Examples of the thio group may include a methylthio group, an ethylthio group, a propylthio group, a pentylthio group, a hexylthio group, an octylthio group, a dodecylthio group, a cyclopentylthio group, a cyclohexylthio group, a phenylthio group, a naphthylthio group, etc., but the present disclosure is not limited to thereto.

In the present description, an alkenyl group may be linear or branched. The number of carbon atoms is not particularly limited, but may be 2 to 30, 2 to 20, or 2 to 10. Examples of the alkenyl group may include a vinyl group, a 1-butenyl group, a 1-pentenyl group, a 1,3-butadienyl group, a styrenyl group, a styryl vinyl group, etc., but the present disclosure is not limited thereto.

In the present description, the number of carbon atoms in an amine group is not particularly limited, but may be 1 to 30. The amine group may include an alkyl amine group and an aryl amine group. Examples of the amine group may include a methylamine group, a dimethylamine group, a phenylamine group, a diphenylamine group, a naphthylamine group, a 9-methyl-anthracenylamine group, etc., but the present disclosure is not limited thereto.

In the present description, an oxy group may refer to a group in which an oxygen atom is bonded to an alkyl group or an aryl group as defined above. The oxy group may include an alkoxy group and an aryl oxy group. The alkoxy group may be linear, branched or cyclic. The number of carbon atoms in the alkoxy group is not particularly limited, but may be, for example, 1 to 20, or 1 to 10. Examples of the oxy group may include a methoxy group, an ethoxy group, an n-propoxy group, an isopropoxy group, a butoxy group, a pentyloxy group, a hexyloxy group, an octyloxy group, a nonyloxy group, a decyloxy group, a benzyloxy group, etc., but the present disclosure is not limited thereto.

In the present description, an alkynyl group may be a hydrocarbon group including at least one carbon triple bond in the middle or terminal end of an alkyl group having 2 or more carbon atoms. The alkynyl group may be a linear chain or a branched chain. The carbon number is not limited, but may be 2 to 30, 2 to 20 or 2 to 10. Examples of the alkynyl group include an ethynyl group, a propynyl group, etc., but the present disclosure is not limited thereto.

In the present description, a boron group may refer to an alkyl boron group or an aryl boron group. Non-limiting examples of the boron group include a dimethylboryl group, a diethylboryl group, a t-butylmethylboryl group, a diphenylboryl group, a phenylboryl group, etc.

In the present description, a phosphine oxide group may be substituted with, for example, at least one of the alkyl groups or aryl groups described above.

In the present description, a phosphine sulfide group may be substituted with, for example, at least one of the alkyl groups or aryl groups described above.

In the present description, the number of ring-forming carbon atoms in the carbonyl group is not specifically limited, but may be 1 to 40, 1 to 30, or 1 to 20. For example, the carbonyl group may have the following structures, but the embodiment of the inventive concept is not limited thereto:

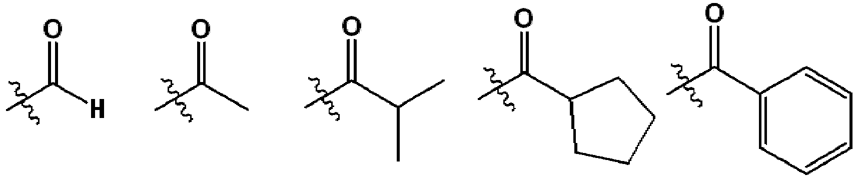

In the present description, the number of carbon atoms in the sulfinyl group and the sulfonyl group is not particularly limited, but may be 1 to 30. The sulfinyl group may include an alkyl sulfinyl group and an aryl sulfinyl group. The sulfonyl group may include an alkyl sulfonyl group and an aryl sulfonyl group.

In the present description, examples of the alkyl group included in an alkylthio group, an alkyl sulfoxy group, an alkylaryl group, an alkylamino group, an alkyl boron group, an alkyl silyl group, and an alkyl amine group may be the same as the examples of the alkyl group described above.

In the present description, examples of the aryl group included in an aryloxy group, an arylthio group, an aryl sulfoxy group, an arylamino group, an aryl boron group, an aryl silyl group, and an aryl amine group may be the same as the examples of the aryl group described above.

In the present description, a direct linkage may refer to a single bond.

Meanwhile, in the present description, "--------∗" refers to a site to be connected.

Hereinafter, embodiments of the present disclosure will be described with reference to the accompanying drawings.

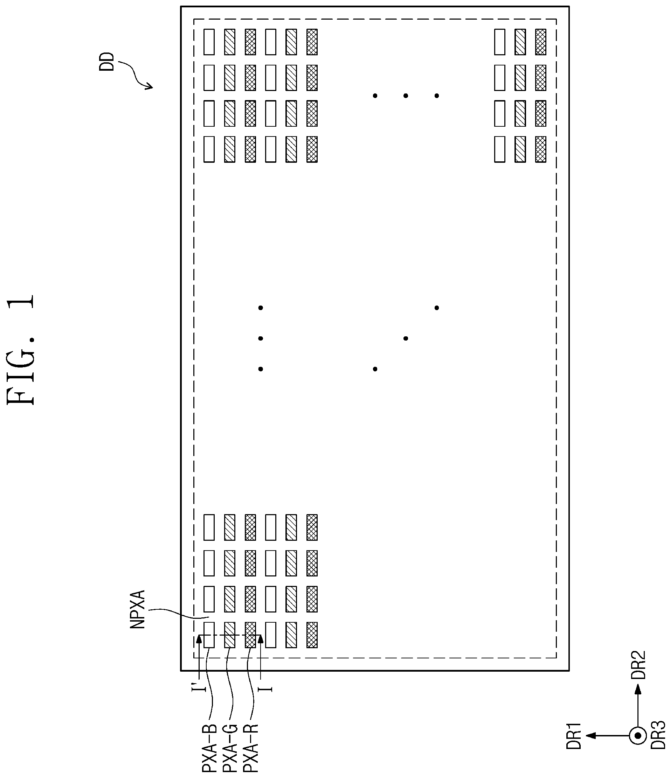

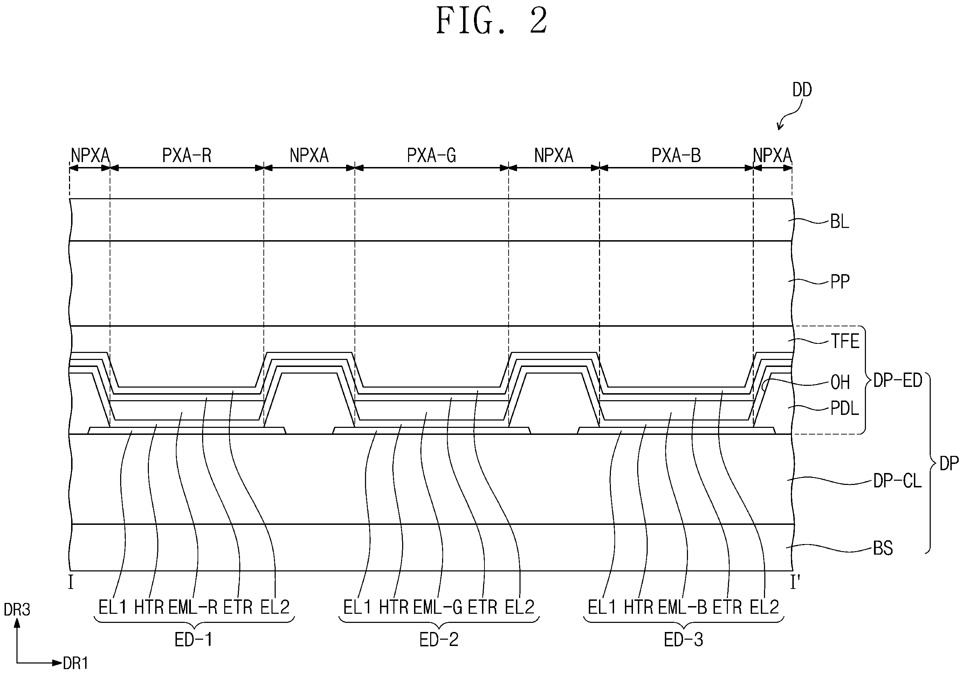

FIG. 1 is a plan view showing an embodiment of a display apparatus DD. FIG. 2 is a cross-sectional view of a display apparatus DD of an embodiment. In particular, FIG. 2 is a cross-sectional view showing a portion corresponding to the line I-I' of FIG. 1.

The display apparatus DD may include a display panel DP and an optical layer PP disposed on the display panel DP. The display panel DP includes luminescence devices ED-1, ED-2, and ED-3. The display apparatus DD may include a plurality of luminescence devices ED-1, ED-2, and ED-3. The optical layer PP may be disposed on the display panel DP to control reflected light in the display panel DP due to external light. The optical layer PP may include, for example, a polarizing layer and/or a color filter layer. Meanwhile, in some embodiments, unlike what is shown in the drawings, the optical layer PP may be omitted in the display apparatus DD of an embodiment.

A base substrate BL may be disposed on the optical layer PP. The base substrate BL may be a member providing a base surface on which the optical layer PP is disposed. The base substrate BL may be a glass substrate, a metal substrate, a plastic substrate, etc. However, the embodiment of the present disclosure is not limited thereto, and the base substrate BL may be an inorganic layer, an organic layer, or a composite material layer (e.g., including an inorganic material and an organic material). In addition, in some embodiments, unlike what is shown, the base substrate BL may be omitted in an embodiment.

The display apparatus DD according to an embodiment may further include a filling layer. The filling layer may be disposed between a display element layer DP-ED and the base substrate BL. The filling layer may be an organic material layer. The filling layer may include at least one selected from among an acrylic (e.g., acrylic-based) resin, a silicone-based resin, and an epoxy-based resin.

The display panel DP may include a base layer BS, a circuit layer DP-CL provided on the base layer BS, and a display element layer DP-ED. The display element layer DP-ED may include pixel defining films PDL, a plurality of luminescence devices ED-1, ED-2, and ED-3 disposed between the pixel defining films PDL, and an encapsulation layer TFE disposed on the plurality of luminescence devices ED-1, ED-2, and ED-3.

The base layer BS may be a member providing a base surface in which the display element layer DP-ED is disposed. The base layer BS may be a glass substrate, a metal substrate, a plastic substrate, etc. However, the embodiment of the present disclosure is not limited thereto, and the base layer BS may be an inorganic layer, an organic layer, or a composite material layer (e.g., including an inorganic material and an organic material).

In an embodiment, the circuit layer DP-CL may be disposed on the base layer BS, and the circuit layer DP-CL may include a plurality of transistors. The transistors may each include a control electrode, an input electrode, and an output electrode. For example, the circuit layer DP-CL may include a switching transistor and a driving transistor for driving a plurality of luminescence devices ED-1, ED-2 and ED-3 of the display element layer DP-ED.

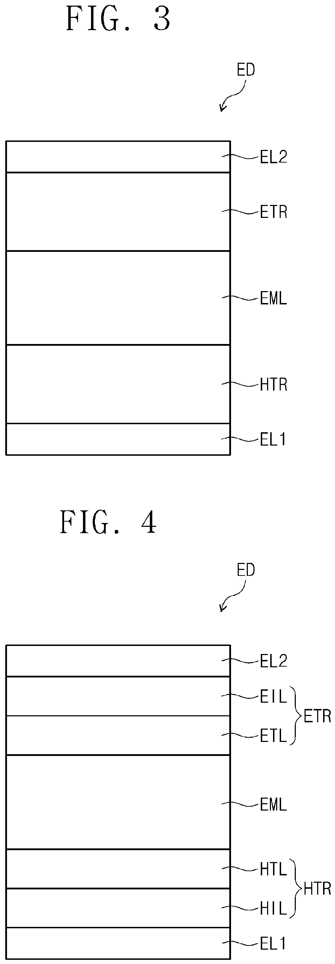

The luminescence devices ED-1, ED-2, and ED-3 each may have a structure of a luminescence device ED of an embodiment shown in each of FIGS. 3 to 6, which will be described in more detail later. The luminescence devices ED-1, ED-2, and ED-3 each may include a first electrode EL1, a hole transport region HTR, one or more of emission layers EML-R, EML-G, and EML-B, an electron transport region ETR, and a second electrode EL2.

FIG. 2 shows an embodiment in which the emission layers EML-R, EML-G, and EML-B of the luminescence devices ED-1, ED-2, and ED-3 are disposed in openings OH defined in the pixel defining films PDL, and the hole transport region HTR, the electron transport region ETR, and the second electrode EL2 are provided as a common layer throughout the luminescence devices ED-1, ED-2, and ED-3. However, the embodiment of the present disclosure is not limited thereto, and unlike what is shown in FIG. 2, in an embodiment, the hole transport region HTR and the electron transport region ETR may be provided to be patterned inside the openings OH defined in the pixel defining films PDL. For example, in an embodiment, the hole transport region HTR, the emission layers EML-R, EML-G, and EML-B, and the electron transport region ETR, etc., of the luminescence devices ED-1, ED-2, and ED-3 may be patterned and provided through an inkjet printing method.

An encapsulation layer TFE may cover the luminescence devices ED-1, ED-2 and ED-3. The encapsulation layer TFE may seal the display element layer DP-ED. The encapsulation layer TFE may be a thin film encapsulation layer. The encapsulation layer TFE may be a single layer or a laminated layer of a plurality of layers. The encapsulation layer TFE includes at least one insulating layer. The encapsulation layer TFE according to an embodiment may include at least one inorganic film (hereinafter, an encapsulation inorganic film). In addition, the encapsulation layer TFE according to an embodiment may include at least one organic film (hereinafter, an encapsulation organic film) and at least one encapsulation inorganic film.

The encapsulation inorganic film protects the display element layer DP-ED from moisture/oxygen, and the encapsulation organic film protects the display element layer DP-ED from foreign substances such as dust particles. The encapsulation inorganic film may include silicon nitride, silicon oxy nitride, silicon oxide, titanium oxide, aluminium oxide, etc., but the present disclosure is not particularly limited thereto. The encapsulation organic layer may include an acrylic (e.g., acrylic-based) compound, an epoxy-based compound, etc. The encapsulation organic layer may include a photopolymerizable organic material, and is not particularly limited.

The encapsulation layer TFE may be disposed on the second electrode EL2, and may be disposed to fill the openings OH.

Referring to FIGS. 1 and 2, the display apparatus DD may include a non-light emitting area NPXA and light emitting areas PXA-R, PXA-G, and PXA-B. The light emitting areas PXA-R, PXA-G, and PXA-B may each be an area emitting light generated from each of the luminescence devices ED-1, ED-2, and ED-3. The light emitting areas PXA-R, PXA-G, and PXA-B may be spaced apart from each other on a plane (e.g., in a plan view).

Each of the light emitting areas PXA-R, PXA-G, and PXA-B may be an area separated by the pixel defining films PDL. The non-light emitting areas NPXA may be an area between neighboring light emitting areas PXA-R, PXA-G, and PXA-B, and may correspond to the pixel defining films PDL. Meanwhile, in the present description, each of the light emitting areas PXA-R, PXA-G, and PXA-B may correspond to a pixel. The pixel defining films PDL may separate the luminescence devices ED-1, ED-2 and ED-3. The emission layers EML-R, EML-G, and EML-B of the luminescence devices ED-1, ED-2 and ED-3 may be disposed and separated in openings OH defined by the pixel defining films PDL.

The light emitting areas PXA-R, PXA-G, and PXA-B may be divided into a plurality of groups according to the color of light generated from the luminescence devices ED-1, ED-2, and ED-3. In the display apparatus DD of an embodiment shown in FIGS. 1 and 2, three light emitting areas PXA-R, PXA-G, and PXA-B which emit red light, green light, and blue light, respectively, are illustrated as an example. For example, the display apparatus DD of an embodiment may include a red light emitting area PXA-R, a green light emitting area PXA-G, and a blue light emitting area PXA-B, which are distinct from one another.

In the display apparatus DD according to an embodiment, the luminescence devices ED-1, ED-2, and ED-3 may emit light having different wavelength ranges. For example, in an embodiment, the display apparatus DD may include a first luminescence device ED-1 emitting red light, a second luminescence device ED-2 emitting green light, and a third luminescence device ED-3 emitting blue light. That is, the red light emitting area PXA-R, the green light emitting area PXA-G, and the blue light emitting area PXA-B of the display apparatus DD may correspond to the first luminescence device ED-1, the second luminescence device ED-2, and the third luminescence device ED-3, respectively.

However, the embodiment of the present disclosure is not limited thereto, and the first to third luminescence devices ED-1, ED-2 and ED-3 may emit light in the same wavelength range or emit light in at least one different wavelength range. For example, the first to third luminescence devices ED-1, ED-2, and ED-3 may all emit blue light.

The light emitting areas PXA-R, PXA-G, and PXA-B in the display apparatus DD according to an embodiment may be arranged in the form of a stripe. Referring to FIG. 1, a plurality of red light emitting areas PXA-R, a plurality of green light emitting areas PXA-G, and a plurality of blue light emitting areas PXA-B may each be arranged along a second directional axis DR2. In some embodiments, the red light emitting area PXA-R, the green light emitting area PXA-G, and the blue light emitting area PXA-B may be alternately arranged in turn along a first directional axis DR1.

FIGS. 1 and 2 show that the light emitting areas PXA-R, PXA-G, and PXA-B are all similar in size, but the embodiment of the present disclosure is not limited thereto, and the light emitting areas PXA-R, PXA-G and PXA-B may be different in size from each other according to the wavelength range of emitted light. In some embodiments, the areas of the light emitting areas PXA-R, PXA-G, and PXA-B may refer to an area when viewed on a plane (e.g., in a plan view) defined by the first directional axis DR1 and the second directional axis DR2.

Meanwhile, the arrangement of the light emitting areas PXA-R, PXA-G, and PXA-B is not limited to what is shown in FIG. 1, and the order in which the red light emitting area PXA-R, the green light emitting area PXA-G, and the blue light emitting area PXA-B are arranged may have varied combination according to display quality characteristics required for the display apparatus DD. For example, the light emitting areas PXA-R, PXA-G, and PXA-B may be arranged in the form of a PENTILE^{®} or a diamond shape.

In addition, an area of each of the light emitting areas PXA-R, PXA-G, and PXA-B may be different in size from one another. For example, in an embodiment, the green light emitting area PXA-G may be smaller than the blue light emitting area PXA-B in size, but the embodiment of the present disclosure is not limited thereto.

Hereinafter, FIGS. 3 to 6 are cross-sectional views schematically showing a luminescence device according to an embodiment. The luminescence device ED according to an embodiment may include a first electrode EL1, a hole transport region HTR, an emission layer EML, an electron transport region ETR, and a second electrode EL2 sequentially stacked.

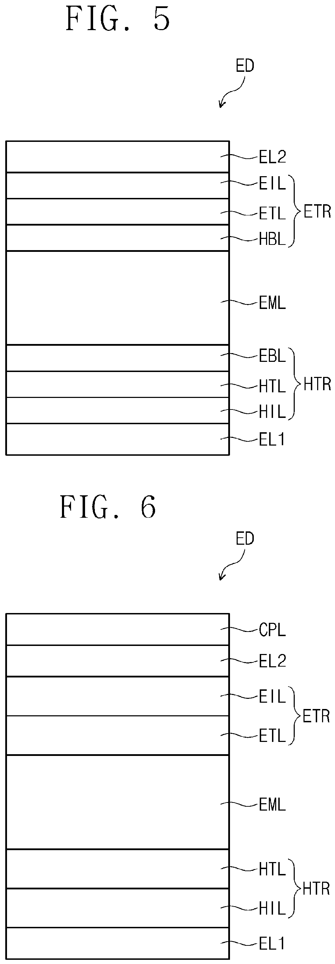

FIG. 4 shows, compared with FIG. 3, a cross-sectional view of a luminescence device ED of an embodiment in which the hole transport region HTR includes a hole injection layer HIL and a hole transport layer HTL, and the electron transport region ETR includes an electron injection layer EIL and an electron transport layer ETL. In addition, FIG. 5 shows, compared with FIG. 3, a cross-sectional view of a luminescence device ED of an embodiment in which the hole transport region HTR includes a hole injection layer HIL, a hole transport layer HTL, and an electron blocking layer EBL, and the electron transport region ETR includes an electron injection layer EIL, an electron transport layer ETL, and a hole blocking layer HBL. FIG. 6 shows, compared with FIG. 4, a cross-sectional view of a luminescence device ED of an embodiment, in which a capping layer CPL disposed on the second electrode EL2 is provided.

The first electrode EL1 has conductivity (e.g., electrical conductivity). The first electrode EL1 may be formed of a metal material, a metal alloy and/or a conductive compound. The first electrode EL1 may be an anode or a cathode. However, the embodiment of the present disclosure is not limited thereto. In addition, the first electrode EL1 may be a pixel electrode. The first electrode EL1 may be a transmissive electrode, a transflective electrode, or a reflective electrode. When the first electrode EL1 is the transmissive electrode, the first electrode EL1 may include a transparent metal oxide such as indium tin oxide (ITO), indium zinc oxide (IZO), zinc oxide (ZnO), and/or indium tin zinc oxide (ITZO). When the first electrode EL1 is the transflective electrode or the reflective electrode, the first electrode EL1 may include Ag, Mg, Cu, Al, Pt, Pd, Au, Ni, Nd, Ir, Cr, Li, Ca, LiF/Ca, LiF/Al, Mo, Ti, W, a compound thereof, or a mixture thereof (e.g., a mixture of Ag and Mg). In some embodiments, the first electrode EL1 may have a multilayer structure including a reflective film or a transflective film formed of the above-described materials, and a transparent conductive film formed of indium tin oxide (ITO), indium zinc oxide (IZO), zinc oxide (ZnO), indium tin zinc oxide (ITZO), etc. For example, the first electrode EL1 may have a three-layer structure of ITO/Ag/ITO, but the present disclosure is not limited thereto. In one or more embodiments, the first electrode EL1 may include the above-described metal materials, a combination of two or more metal materials selected from the above-described metal materials, and/or oxides of the above-described metal materials. The first electrode EL1 may have a thickness of about 700 Å to about 10000 Å. For example, the first electrode EL1 may have a thickness of 1000 Å to about 3000 Å.

The hole transport region HTR may have a single layer formed of a single material, a single layer formed of a plurality of different materials, or a multilayer structure having a plurality of layers formed of a plurality of different materials.

For example, the hole transport region HTR may have a single-layer structure formed of the hole injection layer HIL or the hole transport layer HTL, or a single-layer structure formed of a hole injection material and/or a hole transport material. In some embodiments, the hole transport region HTR may have a single-layer structure formed of a plurality of different materials, or a multi-layer structure in which a hole injection layer HIL/hole transport layer HTL, a hole injection layer HIL/hole transport layer HTL/buffer layer, a hole injection layer HIL/buffer layer, a hole transport layer HTL/buffer layer, or a hole injection layer HIL/hole transport layer HTL/electron blocking layer EBL are stacked in the respective stated order from the first electrode EL1, but the embodiment of the present disclosure is not limited thereto.

The hole transport region HTR of the luminescence device ED of an embodiment includes a nitrogen-containing compound according to an embodiment of the present disclosure.

The nitrogen-containing compound according to an embodiment of the present disclosure is represented by Formula 1 below.

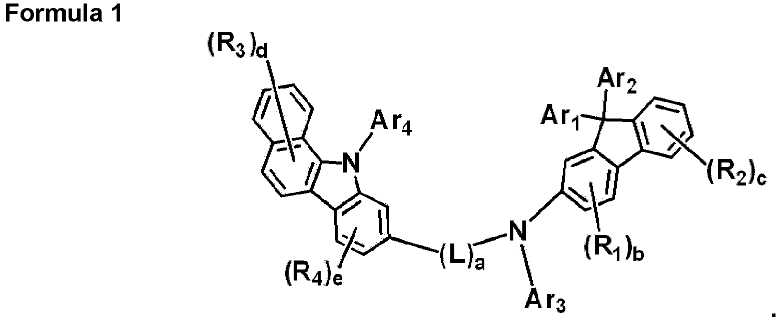

In Formula 1, L is a direct linkage, a substituted or unsubstituted divalent hydrocarbon ring group having 6 to 30 ring-forming carbon atoms, a substituted or unsubstituted arylene group having 6 to 30 ring-forming carbon atoms, a substituted or unsubstituted divalent aliphatic heterocyclic group having 2 to 30 ring-forming carbon atoms, or a substituted or unsubstituted heteroarylene group having 2 to 30 ring-forming carbon atoms.

In an embodiment, L may be a direct linkage or a substituted or unsubstituted arylene group having 6 to 18 ring-forming carbon atoms.

In Formula 1, Ar₁ to Ar₄ are each independently a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In Formula 1, R₁ to R₄ are each independently a hydrogen atom, a deuterium atom, a halogen atom, a nitro group, a cyano group, an oxy group, a substituted or unsubstituted amine group, a substituted or unsubstituted thio group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In Formula 1, a, b, and e are each independently an integer of 0 to 3, wherein when a is 2 or greater, a plurality of L's are the same as or different from each other, when b is 2 or greater, a plurality of R₁'s are the same as or different from each other, and when e is 2 or greater, a plurality of R₄'s are the same as or different from each other.

In Formula 1, c is an integer of 0 to 4, wherein when c is 2 or greater, a plurality of R₂'s are the same as or different from each other.

In Formula 1, d is an integer of 0 to 6, wherein when d is 2 or greater, a plurality of R₃'s are the same as or different from each other.

In an embodiment, Ar₁ to Ar₄ may be each independently a substituted or unsubstituted aryl group having 6 to 30 ring-forming carbon atoms.

In an embodiment, the nitrogen-containing compound represented by Formula 1 may be represented by Formula 2-1 or Formula 2-2 below.

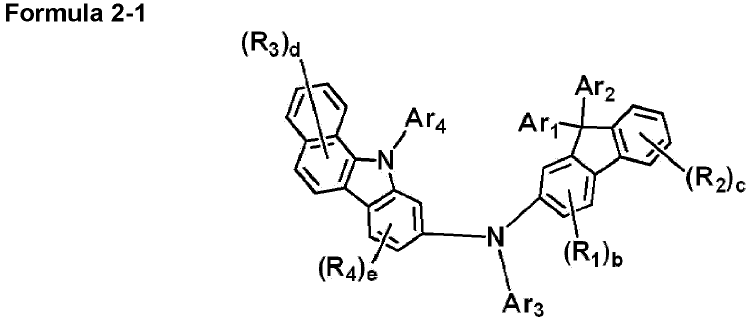

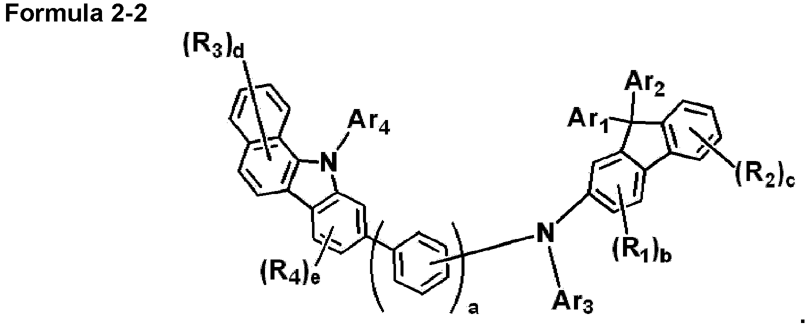

In Formulae 2-1 and 2-2, Ar₁ to Ar₄, R₁ to R₄, and a to e are the same as respectively defined in Formula 1.

In an embodiment, the nitrogen-containing compound represented by Formula 1 may be represented by Formula 3 below.

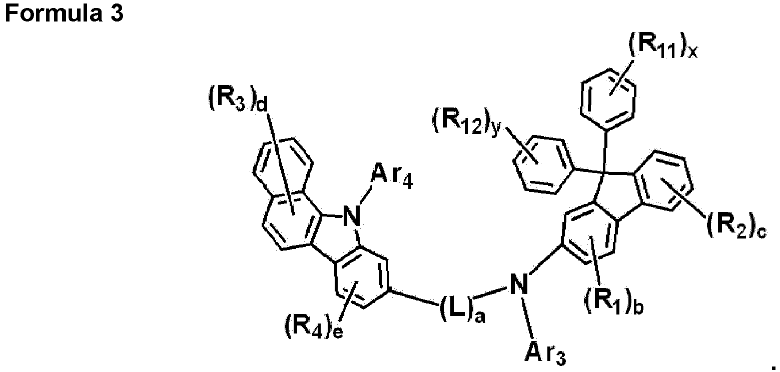

In Formula 3, R₁₁ and R₁₂ are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring,

In Formula 3, x and y are each independently an integer of 0 to 5, wherein when x is 2 or greater, a plurality of R₁₁'s are the same as or different from each other, and when y is 2 or greater, a plurality of R₁₂'s are the same as or different from each other.

In Formula 3, Ar₃, Ar₄, R₁ to R₄, L, and a to e are the same as respectively defined in Formula 1.

In an embodiment, the nitrogen-containing compound represented by Formula 3 may be represented by Formula 5 below.

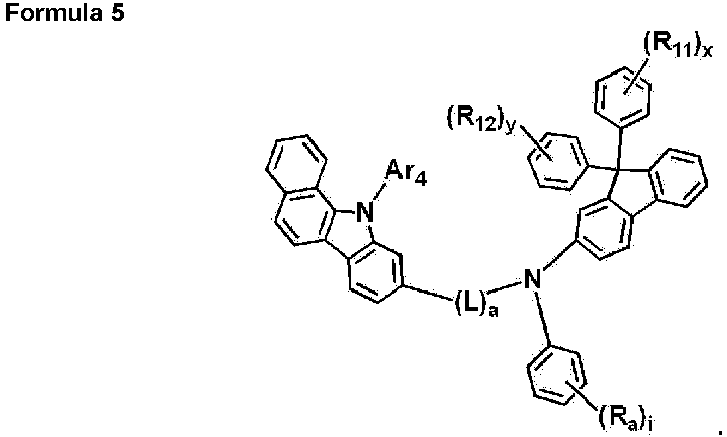

In Formula 5, Rₐ is a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring.

In Formula 5, i is an integer of 0 to 5, wherein when i is 2 or greater, a plurality of Rₐ's are the same as or different from each other.

In Formula 5, Ar₄, R₁₁, R₁₂, L, a, x, and y are the same as respectively defined in Formula 3.

In an embodiment, the nitrogen-containing compound represented by Formula 1 may be a monoamine compound. The nitrogen-containing compound of the present disclosure may not include an amine group other than the amine group represented in Formula 1. For example, Ar₁ to Ar₄, and R₁ to R₄ may not include an amine group.

In an embodiment, Ar₃ and Ar₄ may be each independently a substituted or unsubstituted aryl group having 6 to 30 ring-forming carbon atoms. In an embodiment, Ar₃ and Ar₄ may be each independently represented by any one of Formulas 4-1 to 4-4 below.

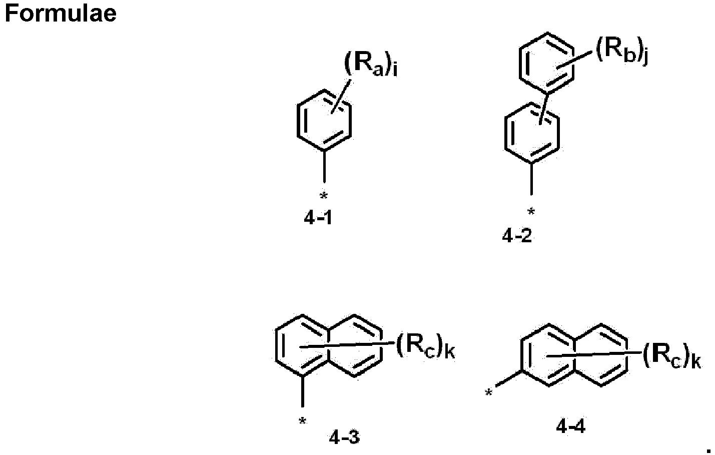

In Formulae 4-1 to 4-4, Rₐ, R_{b} and R_{c} are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring.

In Formulae 4-1 to 4-4, i and j are each independently an integer of 0 to 5. In some embodiments, when i is 2 or greater, a plurality of Rₐ's are the same as or different from each other, and when j is 2 or greater, a plurality of R_{b}'s are the same as or different from each other.

In Formulae 4-1 to 4-4, k is an integer of 0 to 7. In some embodiments, when k is 2 or greater, a plurality of R_{c}'s may be the same as or different from each other.

The nitrogen-containing compound represented by Formula 1 according to an embodiment of the present disclosure may be any one selected from compounds represented by Compound Group 1 below. However, the embodiment of the present disclosure is not limited thereto.

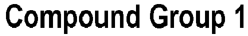

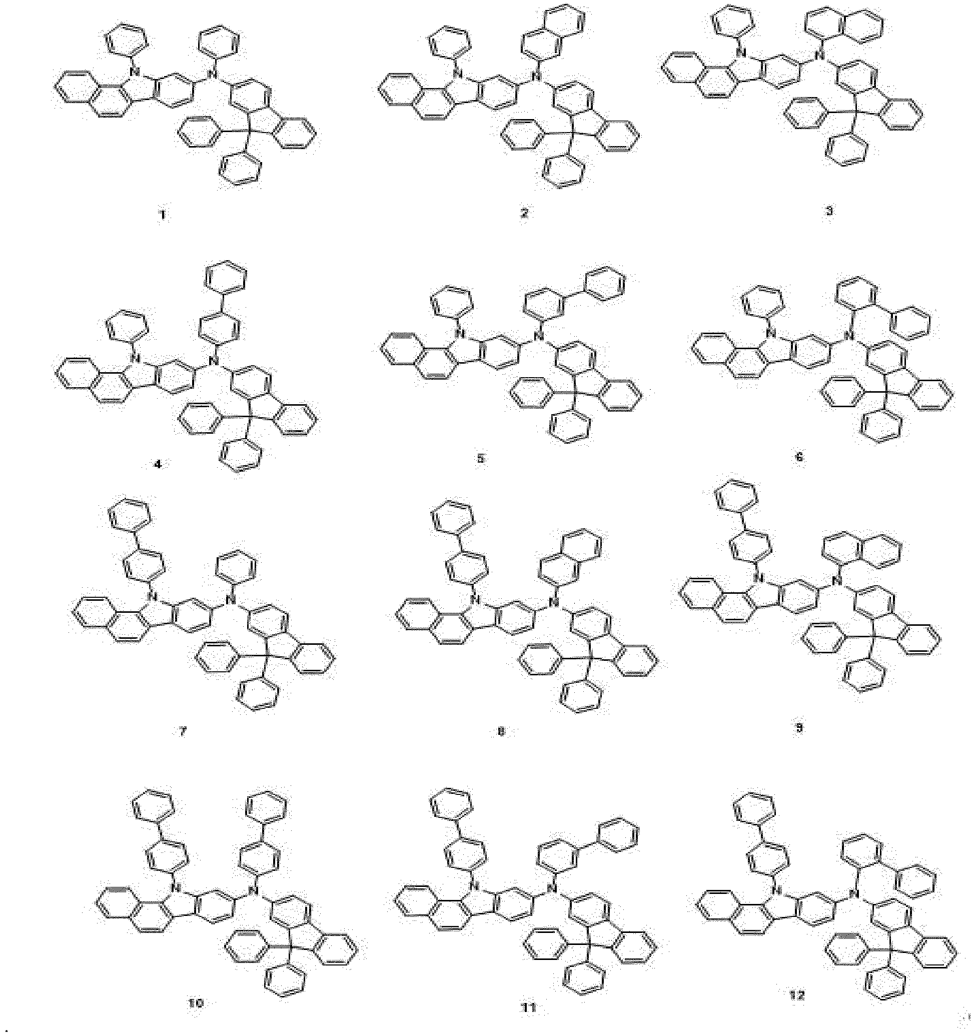

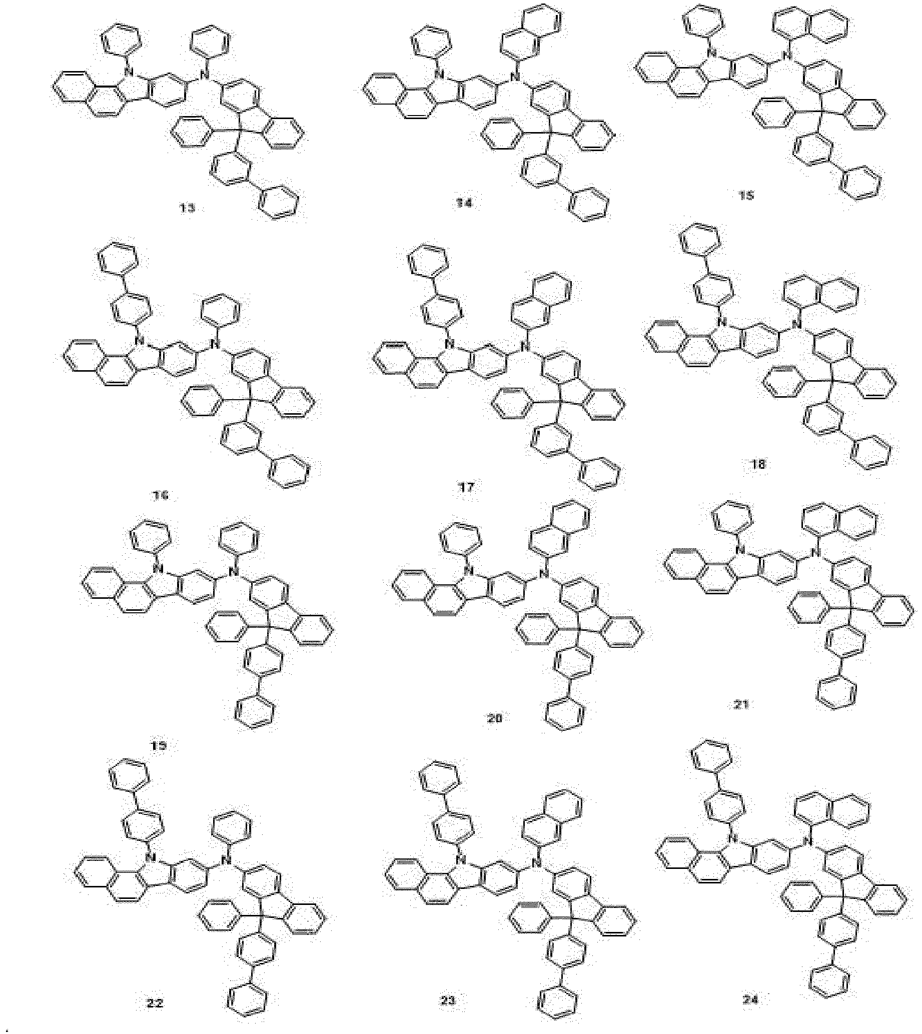

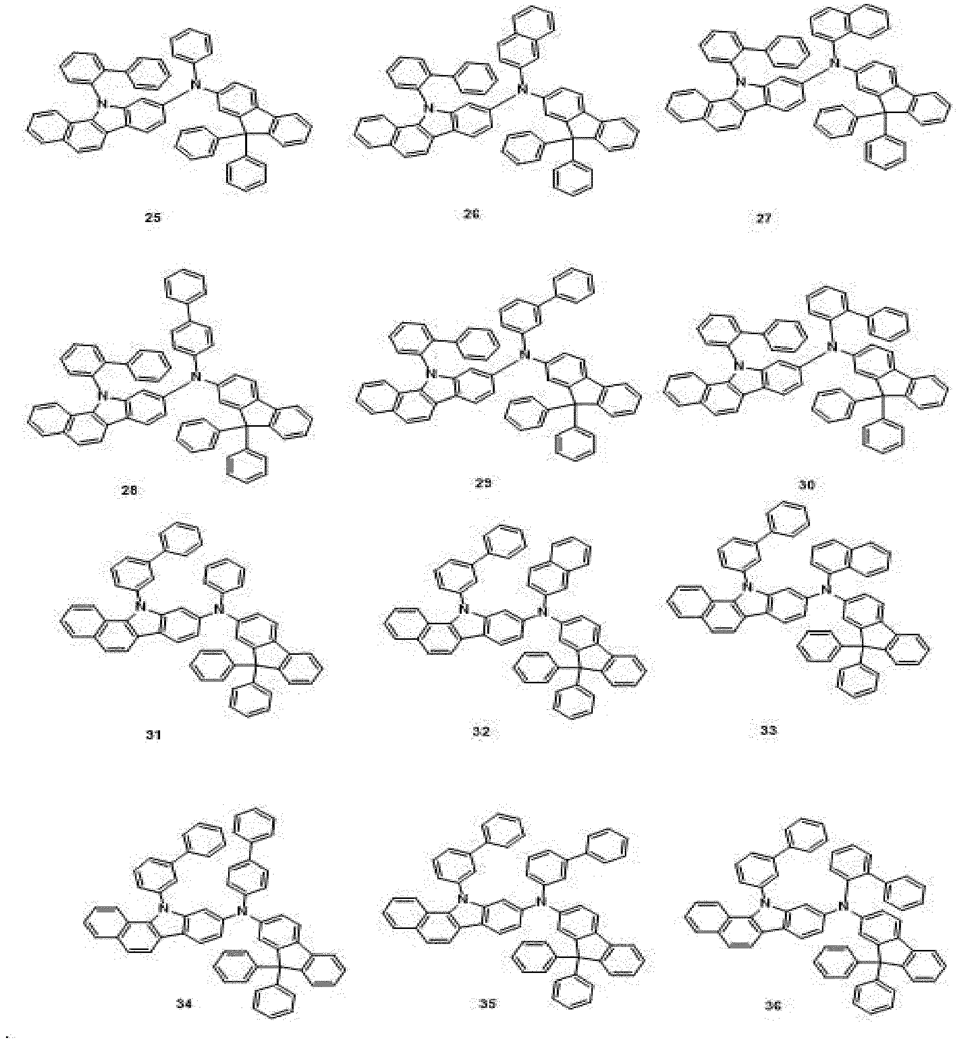

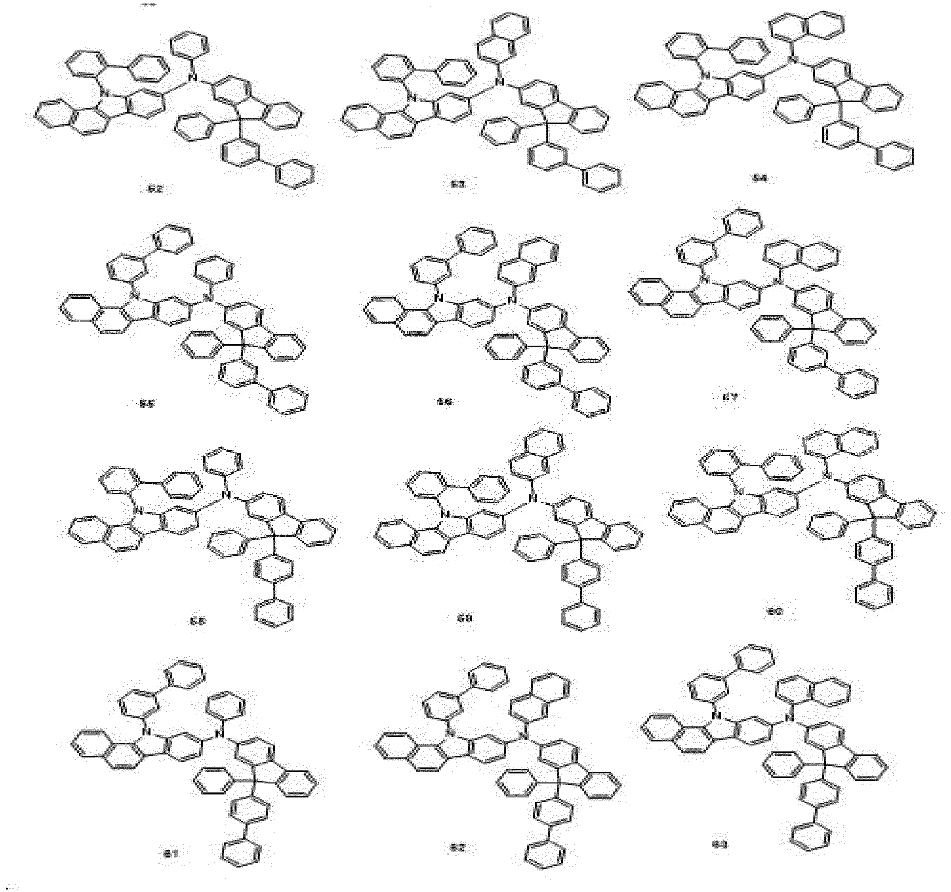

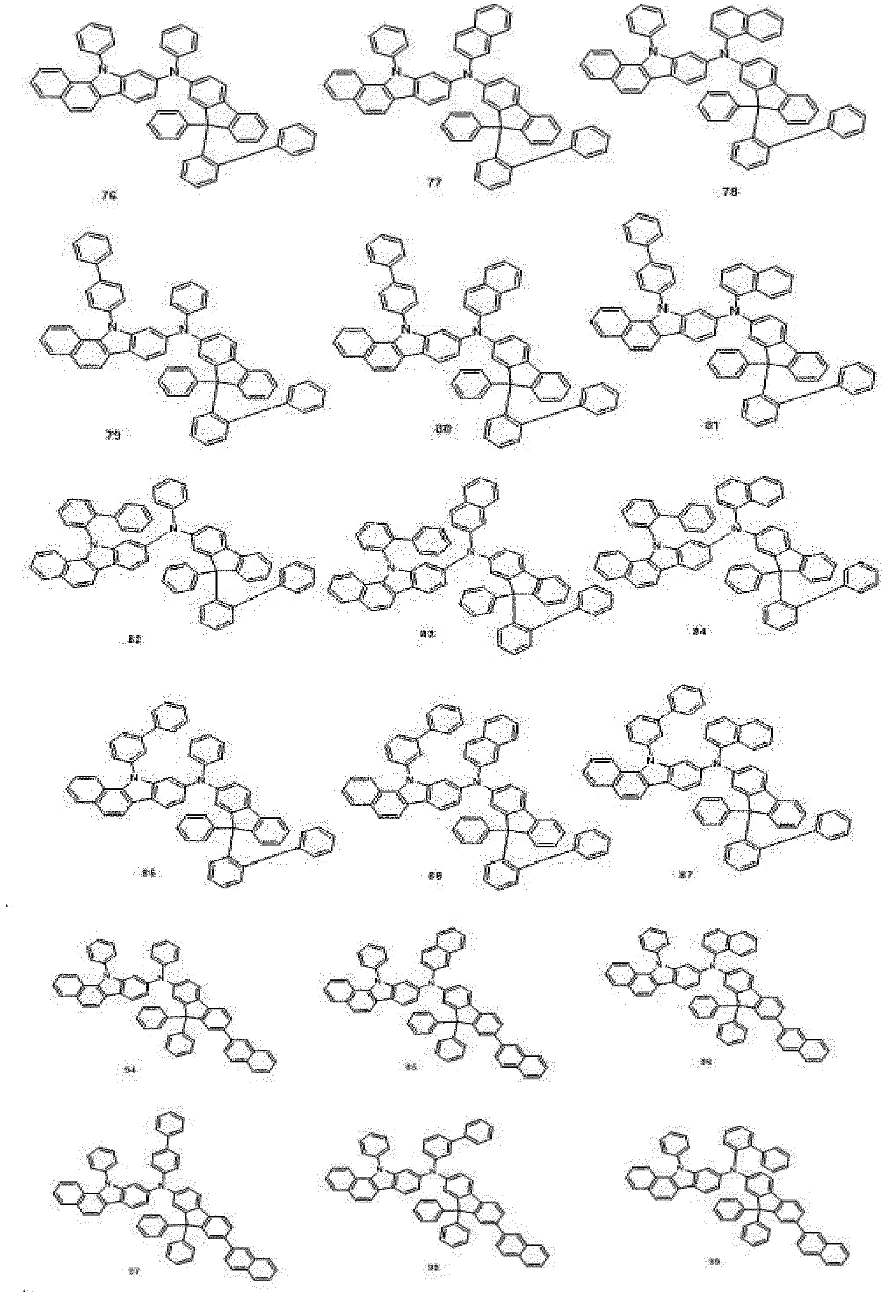

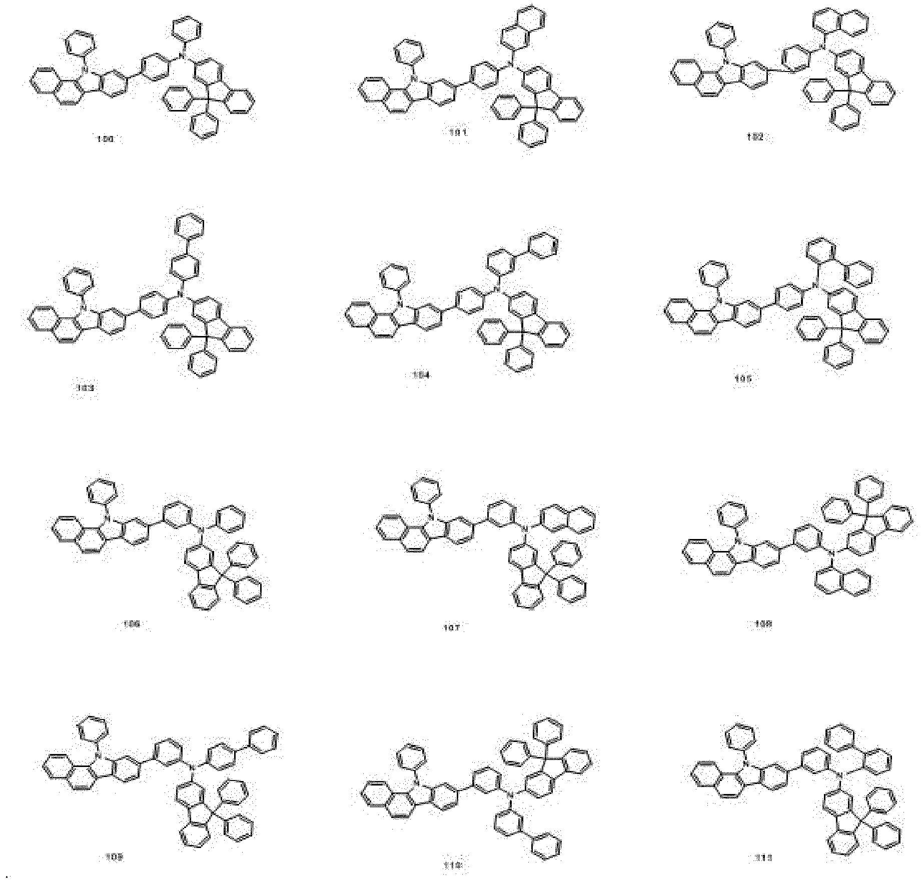

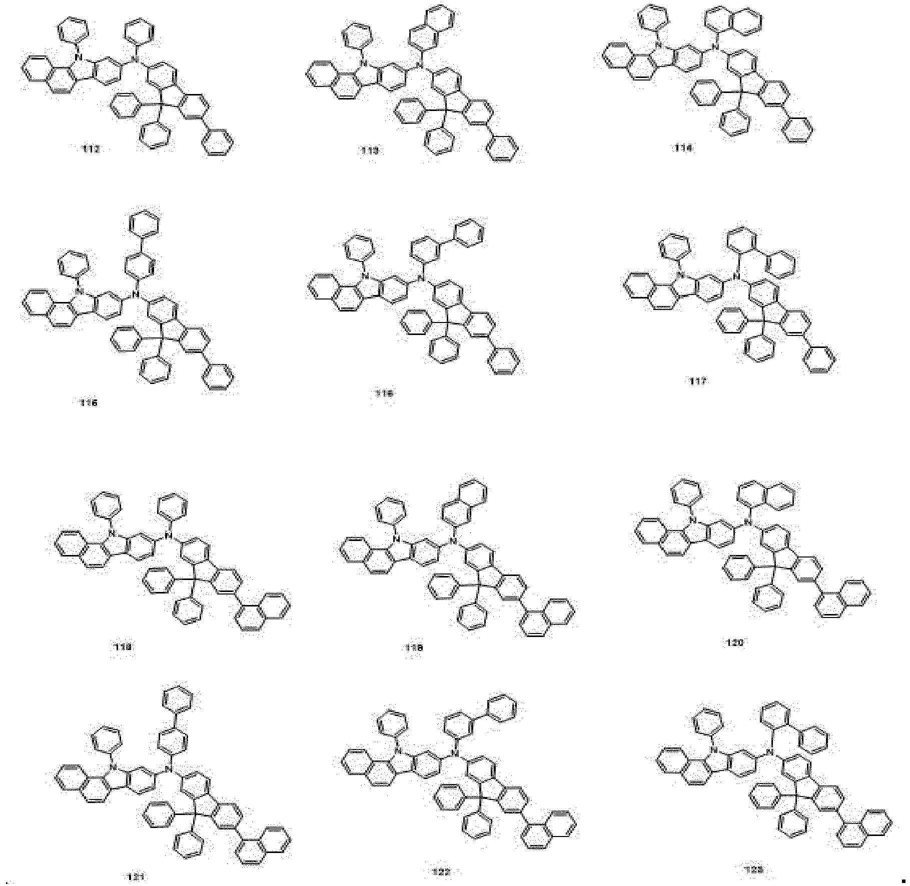

Referring back to FIGS. 3 to 6, the luminescence device ED according to an embodiment of the present disclosure will be described in more detail.

As described above, the hole transport region HTR includes a nitrogen-containing compound according to an embodiment of the present disclosure. For example, the hole transport region HTR includes a nitrogen-containing compound represented by Formula 1.

When the hole transport region HTR has a multilayer structure having a plurality of layers, any one of the plurality of layers may include the nitrogen-containing compound represented by Formula 1. For example, the hole transport region HTR may include a hole injection layer HIL disposed on the first electrode EL1 and a hole transport layer HTL disposed on the hole injection layer HIL, and the hole transport layer HTL may include the nitrogen-containing compound represented by Formula 1. However, the embodiment of the present disclosure is not limited thereto, and for example, the hole injection layer HIL may include the nitrogen-containing compound represented by Formula 1.

The hole transport region HTR may include one, two, or more nitrogen-containing compounds represented by Formula 1. For example, the hole transport region HTR may include at least one selected from the compounds represented by Compound Group 1 described above.

The hole transport region HTR may be formed utilizing various suitable methods such as a vacuum deposition method, a spin coating method, a cast method, a Langmuir-Blodgett (LB) method, an inkjet printing method, a laser printing method, and/or a laser induced thermal imaging (LITI) method.

The hole transport region HTR may further include a compound represented by Formula H-1 below.

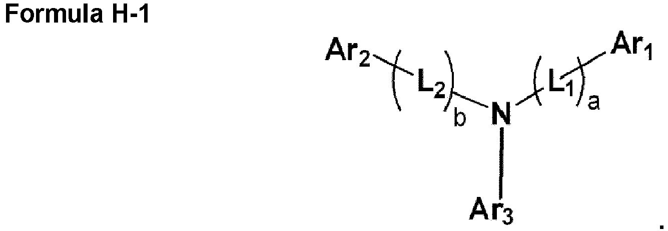

In Formula H-1 above, L₁ and L₂ are each independently a direct linkage, a substituted or unsubstituted arylene group having 6 to 30 ring-forming carbon atoms, or a substituted or unsubstituted heteroarylene group having 2 to 30 ring-forming carbon atoms. a and b are each independently an integer of 0 to 10. In some embodiments, when a and/or b is an integer of 2 or greater, a plurality of L₁'s and/or L₂'s may be each independently a substituted or unsubstituted arylene group having 6 to 30 ring-forming carbon atoms, or a substituted or unsubstituted heteroarylene group having 2 to 30 ring-forming carbon atoms.

In Formula H-1, Ar₁ and Ar₂ are each independently a substituted or unsubstituted aryl group having 6 to 30 ring-forming carbon atoms, or a substituted or unsubstituted heteroaryl group having 2 to 30 ring-forming carbon atoms. In addition, in Formula H-1, Ar₃ is a substituted or unsubstituted aryl group having 6 to 30 ring-forming carbon atoms.

In some embodiments, a compound represented by Formula H-1 above may be a monoamine compound. In some embodiments, the compound represented by Formula H-1 may be a diamine compound in which at least one of Ar₁ to Ar₃ includes an amine group as a substituent. In addition, the compound represented by Formula H-1 may be a carbazole-based compound including a substituted or unsubstituted carbazole group in at least one of Ar₁ or Ar₂, or a fluorene-based compound including a substituted or unsubstituted fluorene group in at least one of Ar₁ or Ar₂.

The compound represented by Formula H-1 may be represented by any one of the compounds from Compound Group H below. However, the compounds listed in Compound Group H below are presented as an example, and the compound represented by Formula H-1 is not limited to those listed in Compound Group H below.

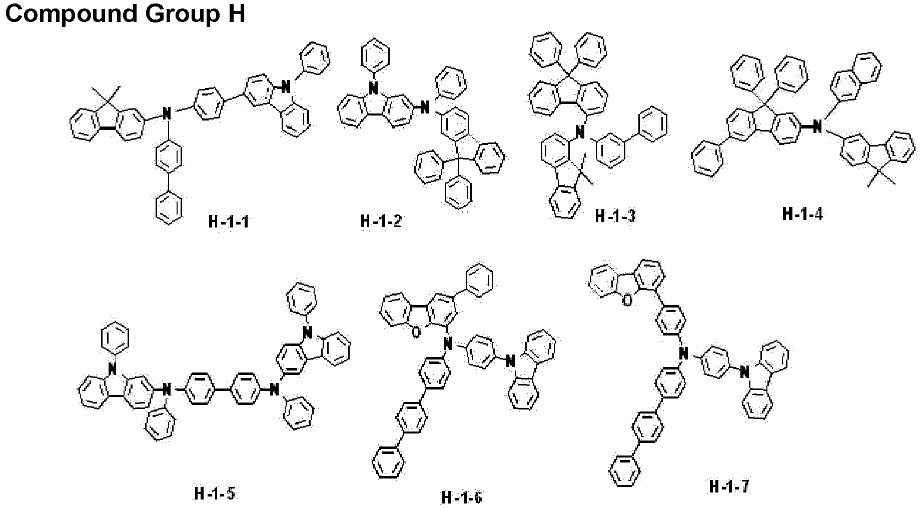

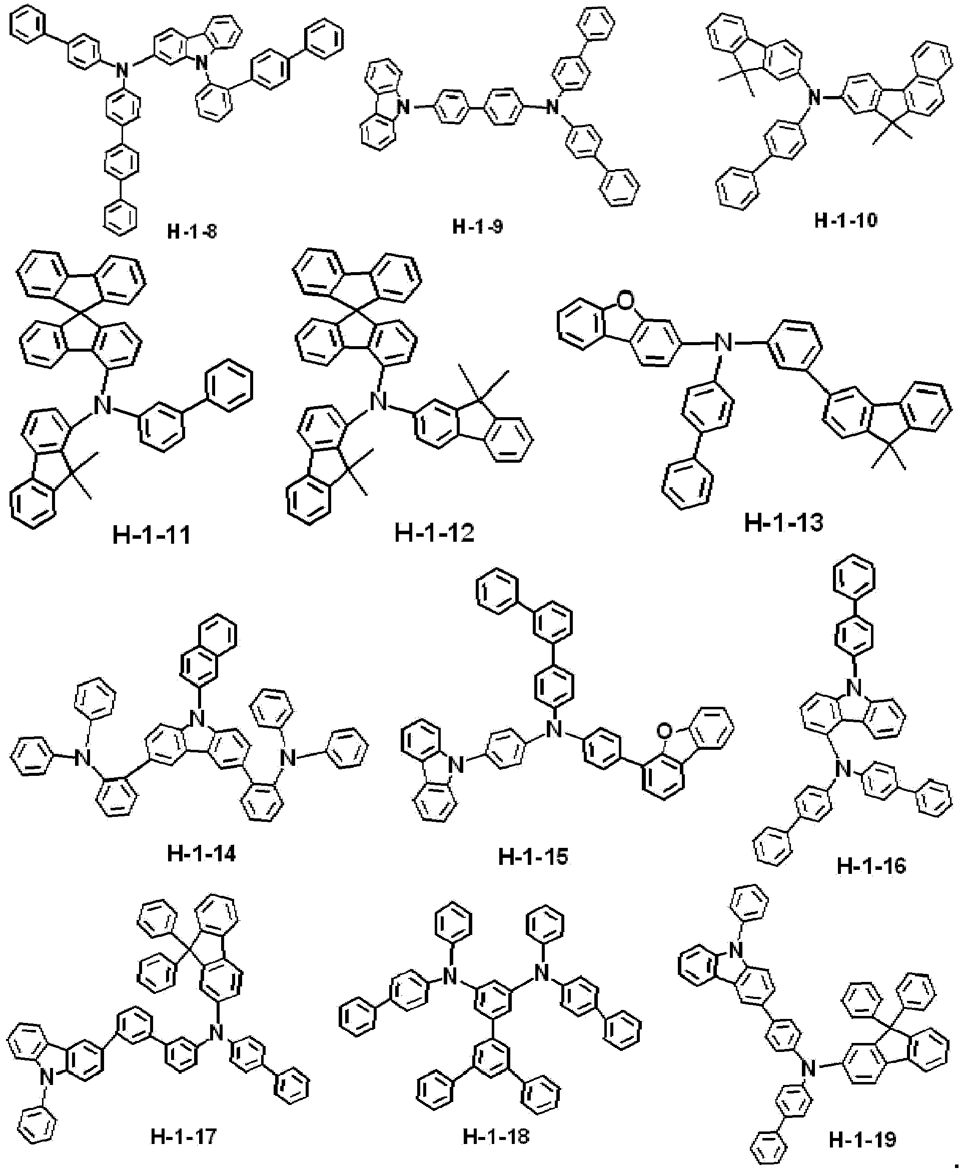

The hole transport region HTR may include a phthalocyanine compound such as copper phthalocyanine, N,N'-diphenyl-N,N'-bis-[4-(phenyl-m-tolyl-amino)-phenyl]-biphenyl-4,4'-diamine (DNTPD), 4,4',4"-[tris(3-methylphenyl)phenylamino]triphenylamine (m-MTDATA), 4,4',4"-tris(N,N-diphenylamino)triphenylamine (TDATA), 4,4',4"-tris[N(1 -naphthyl)-N-phenylamino]-triphenylamine (1 -TNATA), 4,4',4"-tris[N(2-naphthyl)-N-phenylamino]-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonic acid (PANI/CSA), polyaniline/poly(4-styrenesulfonate) (PANI/PSS), N,N'-di(naphthalene-l-yl)-N,N'-diphenyl-benzidine (NPB), triphenylamine-containing polyetherketone (TPAPEK), 4-isopropyl-4'-methyldiphenyliodonium tetrakis(pentafluorophenyl)borate, dipyrazino[2,3-f: 2',3'-h]quinoxaline-2,3,6,7,10,11-hexacarbonitrile (HAT-CN), etc.

The hole transport region HTR may include one or more carbazole-based derivatives such as N-phenyl carbazole and/or polyvinyl carbazole, fluorene-based derivatives, N,N'-bis(3-methylphenyl)-N,N'-diphenyl-[1,1'-biphenyl]-4,4'-diamine (TPD), triphenylamine-based derivatives such as 4,4',4"-tris(N-carbazolyl)triphenylamine (TCTA), N,N'-di(1-naphthalene-1-yl)-N,N'-diphenyl-benzidine (NPB), 4,4'-cyclohexylidene bis[N,N-bis(4-methylphenyl)benzenamine] (TAPC), 4,4'-bis[N,N'-(3-tolyl)amino]-3,3'-dimethylbiphenyl (HMTPD), 1,3-bis(N-carbazolyl)benzene (mCP), etc.

The hole transport region HTR may further include 9-(4-tert-butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol-9-yl)benzene (mDCP), etc.

The hole transport region HTR may include the compounds of the hole transport region HTR described above in at least one among the hole injection layer HIL, the hole transport layer HTL, and the electron blocking layer EBL.

The hole transport region HTR may have a thickness of about 100 Å to about 10000 Å, for example, about 100 Å to about 5000 Å. The hole injection layer HIL, for example, may have a thickness of about 30 Å to about 1000 Å, and the hole transport layer HTL may have a thickness of about 30 Å to about 1000 Å. For example, the electron blocking layer EBL may have a thickness of about 10 Å to about 1000 Å. When the thicknesses of the hole transport region HTR, the hole injection layer HIL, the hole transport layer HTL, and the electron blocking layer EBL satisfy the above-described respective ranges, satisfactory hole transport properties may be obtained without a substantial increase in driving voltage.

The hole transport region HTR may further include, in addition to the above-described materials, a charge generation material to increase conductivity. The charge generation material may be uniformly or non-uniformly dispersed in the hole transport region HTR. The charge generation material may be, for example, a p-dopant. The p-dopant may be one of quinone derivatives, metal oxides, or cyano group-containing compounds, but the present disclosure is not limited thereto. Non-limiting examples of the p-dopant may include quinone derivatives such as tetracyanoquinodimethane (TCNQ) and/or 2,3,5,6-tetrafluoro-7,7,8,8-tetracyanoquinodimethane (F4-TCNQ), metal oxides such as tungsten oxides and/or molybdenum oxides, etc., but the present disclosure is not limited thereto.

As described above, the hole transport region HTR may further include at least one of a hole buffer layer or an electron blocking layer EBL in addition to the hole injection layer HIL and the hole transport layer HTL. The hole buffer layer may compensate a resonance distance according to the wavelength of light emitted from an emission layer EML, and may thus increase luminous efficiency. Materials which may be included in the hole transport region HTR may be utilized as materials included in the hole buffer layer. The electron blocking layer EBL is a layer that serves to prevent or substantially prevent electrons from being injected from the electron transport region ETR to the hole transport region HTR.

The emission layer EML is provided on the hole transport region HTR. The emission layer EML may have, for example, a thickness of about 100 Å to about 1000 Å or about 100 Å to about 300 Å. The emission layer EML may have a single layer formed of a single material, a single layer formed of a plurality of different materials, or a multilayer structure having a plurality of layers formed of a plurality of different materials.

In the luminescence device ED of an embodiment, the emission layer EML may include an anthracene derivative, a pyrene derivative, a fluoranthene derivative, a chrysene derivative, a dihydrobenzanthracene derivative, and/or a triphenylene derivative. For example, the emission layer EML may include an anthracene derivative and/or a pyrene derivative.

In the luminescence device ED of the embodiment shown in FIGS. 3 to 6, the emission layer EML may include a host and a dopant, and the emission layer EML may include a compound represented by Formula E-1 below. The compound represented by Formula E-1 below may be utilized as a fluorescent host material.

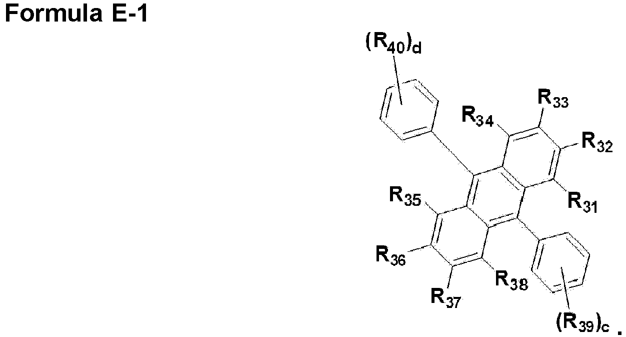

In Formula E-1, R₃₁ to R₄₀ are each independently a hydrogen atom, a deuterium atom, a halogen atom, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms; or bonded to an adjacent group to form a ring. In some embodiments, R₃₁ to R₄₀ may be bonded to an adjacent group to form a saturated hydrocarbon ring, an unsaturated hydrocarbon ring, a saturated heterocycle, or an unsaturated heterocycle.

In Formula E-1, c and d are each independently an integer of 0 to 5.

The compound represented by Formula E-1 may be represented by any one among compounds E1 to E19 below.

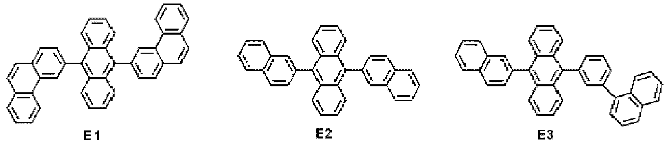

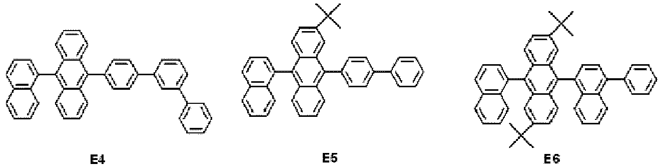

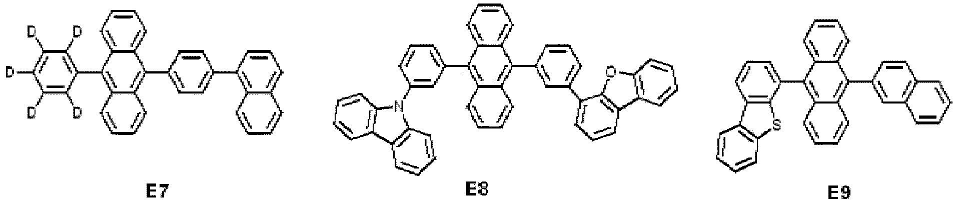

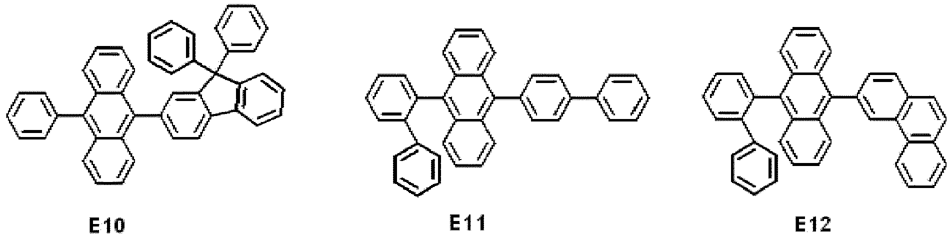

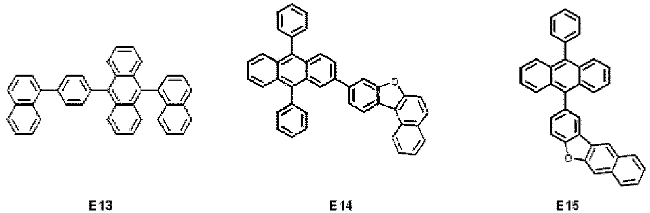

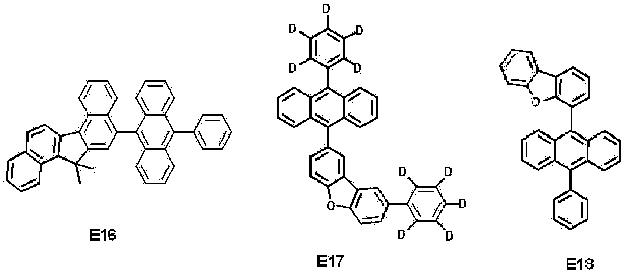

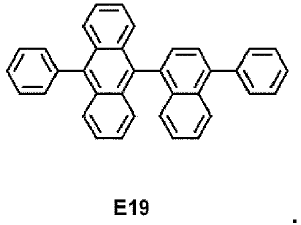

In an embodiment, the emission layer EML may include a compound represented by Formula E-2a or Formula E-2b below. The compound represented by Formula E-2a or Formula E-2b may be utilized as a phosphorescent host material.

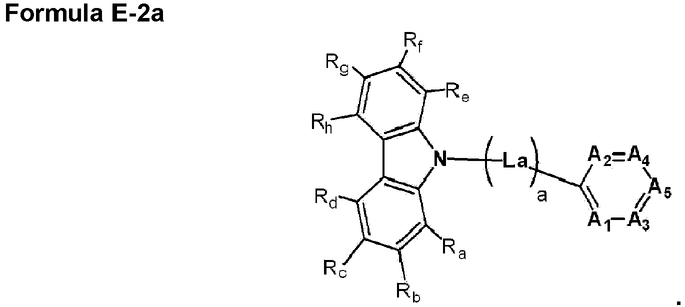

In Formula E-2a, a is an integer of 0 to 10, and Lₐ is a direct linkage, a substituted or unsubstituted arylene group having 6 to 30 ring-forming carbon atoms, or a substituted or unsubstituted heteroarylene group having 2 to 30 ring-forming carbon atoms. In some embodiments, when a is an integer of 2 or greater, a plurality of Lₐ's may be each independently a substituted or unsubstituted arylene group having 6 to 30 ring-forming carbon atoms, or a substituted or unsubstituted heteroarylene group having 2 to 30 ring-forming carbon atoms.

In addition, in Formula E-2a, A₁ to A₅ are each independently N or CRᵢ. Rₐ to Rᵢ are each independently a hydrogen atom, a deuterium atom, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring. Rₐ to Rᵢ may be bonded to an adjacent group to form a hydrocarbon ring or a heterocycle containing N, O, S, etc. as a ring-forming atom.

In some embodiments, in Formula E-2a, two or three selected from A₁ to A₅ may be N, and the rest (e.g., the remainder thereof) may each independently be CRᵢ.

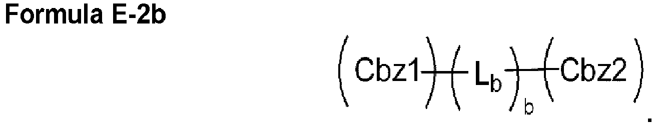

In Formula E-2b, Cbz1 and Cbz2 are each independently an unsubstituted carbazole group or an aryl-substituted carbazole group having 6 to 30 ring-forming carbon atoms. L_{b} is a direct linkage, a substituted or unsubstituted arylene group having 6 to 30 ring-forming carbon atoms, or a substituted or unsubstituted heteroarylene group having 2 to 30 ring-forming carbon atoms, b is an integer of 0 to 10, and when b is an integer of 2 or greater, a plurality of L_{b}'s may be each independently a substituted or unsubstituted arylene group having 6 to 30 ring-forming carbon atoms, or a substituted or unsubstituted heteroarylene group having 2 to 30 ring-forming carbon atoms.

The compound represented by Formula E-2a or Formula E-2b may be represented by any one of the compounds from Compound Group E-2 below. However, the compounds listed in Compound Group E-2 below are presented as an example, and the compound represented by Formula E-2a or Formula E-2b is not limited to those listed in Compound Group E-2 below.

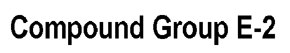

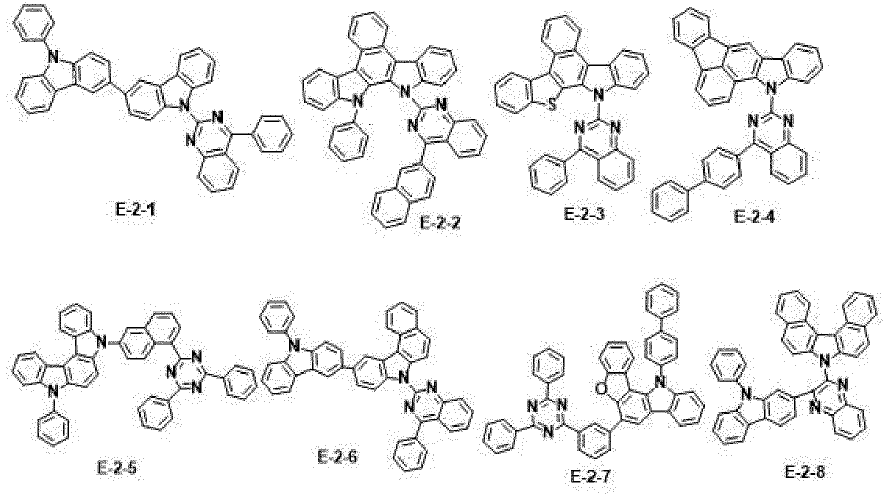

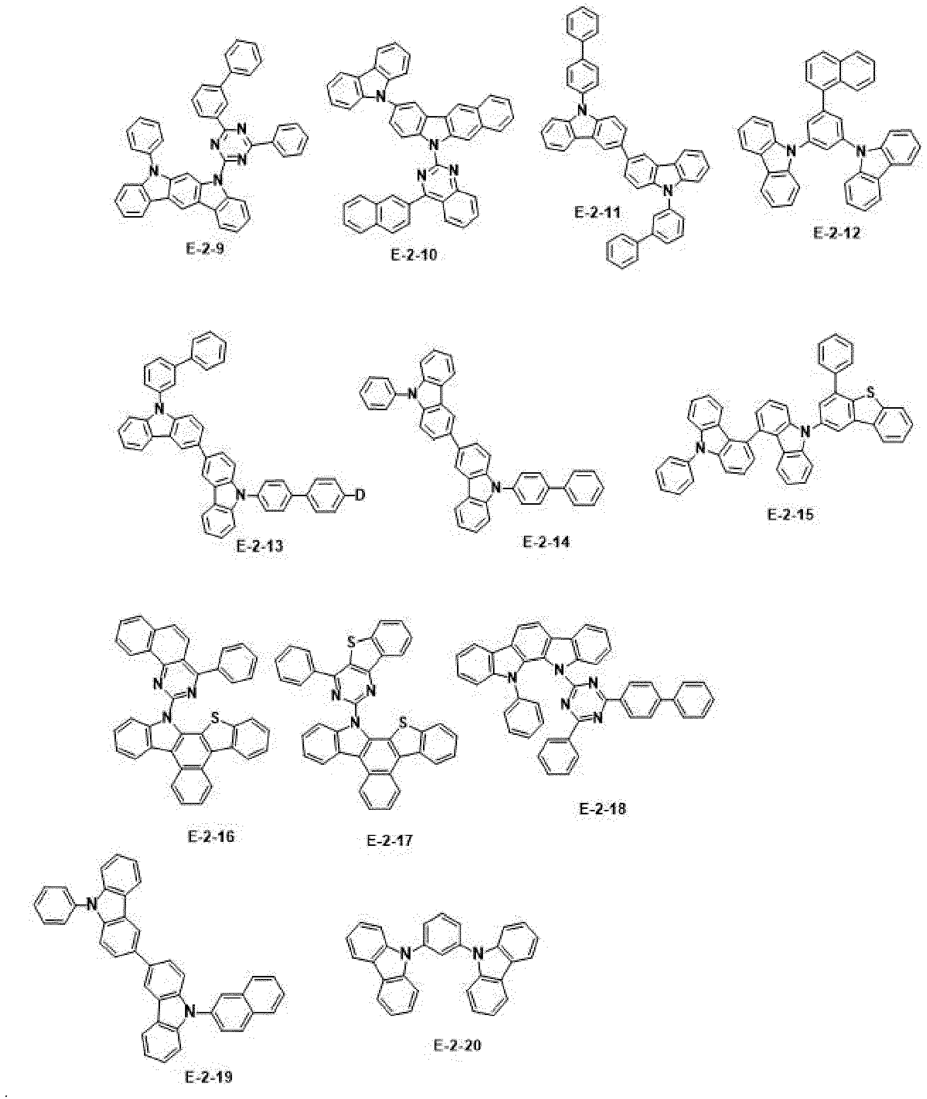

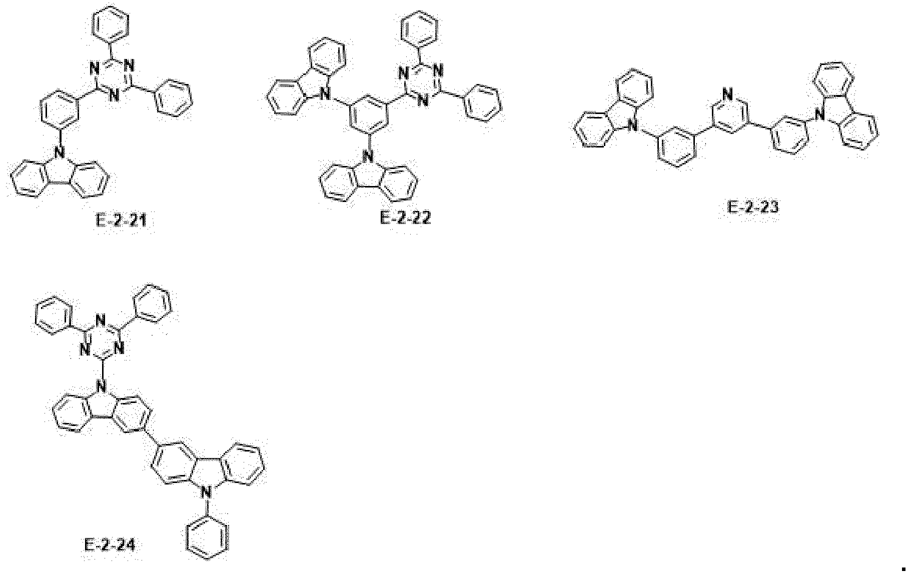

The emission layer EML may further include a general material known in the art as a host material. For example, the emission layer EML may include, as a host material, at least one among bis(4-(9H-carbazol-9-yl)phenyl)diphenylsilane (BCPDS), (4-(1-(4-(diphenylamino)phenyl)cyclohexyl)phenyl)diphenyl-phosphine oxide (POPCPA), bis[2-(diphenylphosphino)phenyl]ether oxide (DPEPO), 4,4'-bis(N-carbazolyl)-1,1-biphenyl (CBP), 1,3-bis(carbazolyl-9-yl)benzene (mCP), 2,8-bis(diphenylphosphoryl)dibenzofuran (PPF), 4,4',4"-tris(carbazol-9-yl)-triphenylamine (TCTA), and 1,3,5-tris(1-phenyl-1H-benzo[d]imidazol-2-yl)benzene (TPBi). However, the embodiment of the present disclosure is not limited thereto, and for example, tris(8-hydroxyquinolino)aluminium (Alq₃), 9,10-di(naphthalene-2-yl)anthracene (ADN), 3-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH2), hexaphenylcyclotrisiloxane (DPSiO₃), octaphenylcyclotetrasiloxane (DPSiO₄), etc. may be utilized as a host material.

The emission layer EML may include a compound represented by Formula M-a or Formula M-b below. The compound represented by Formula M-a or Formula M-b below may be utilized as a phosphorescent dopant material.

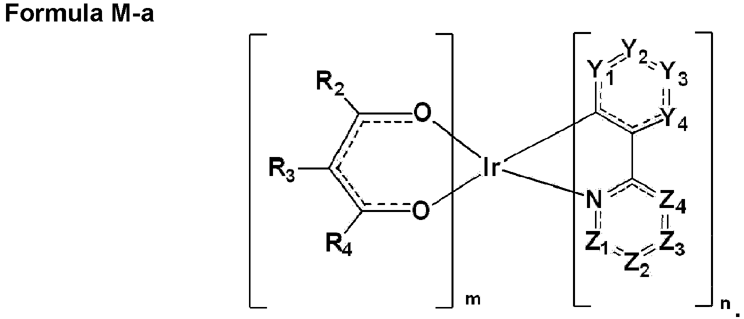

In Formula M-a above, Y₁ to Y₄, and Z₁ to Z₄ are each independently CR₁ or N, and R₁ to R₄ are each independently a hydrogen atom, a deuterium atom, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring. In Formula M-a, m is 0 or 1, and n is 2 or 3. In Formula M-a, when m is 0, n is 3, and when m is 1, n is 2.

The compound represented by Formula M-a may be utilized as a phosphorescent dopant.

The compound represented by Formula M-a may be represented by any one of the compounds M-a1 to M-a25 below. However, the compounds M-a1 to M-a25 below are presented as an example, and the compound represented by Formula M-a is not limited to those represented by the compounds M-a1 to M-a25 below.

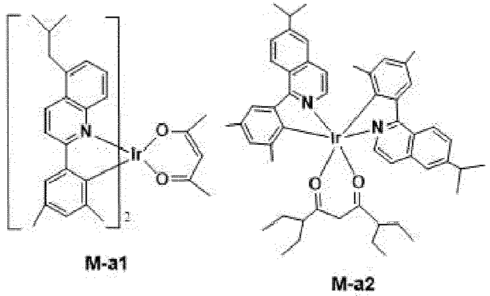

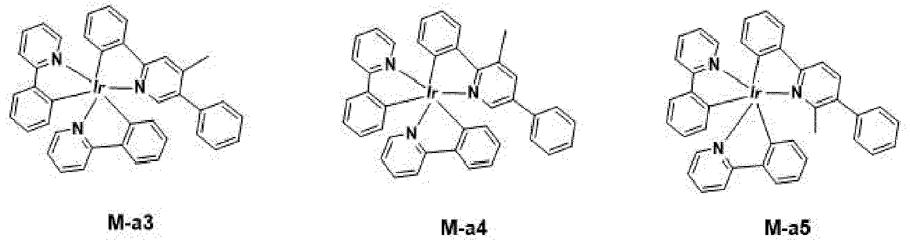

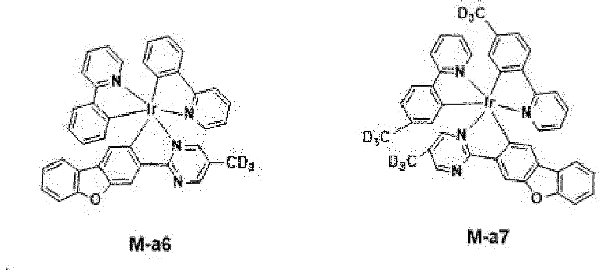

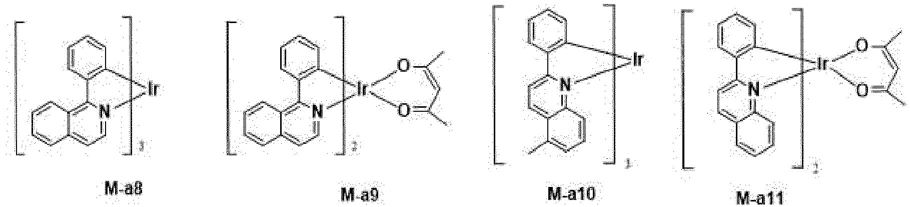

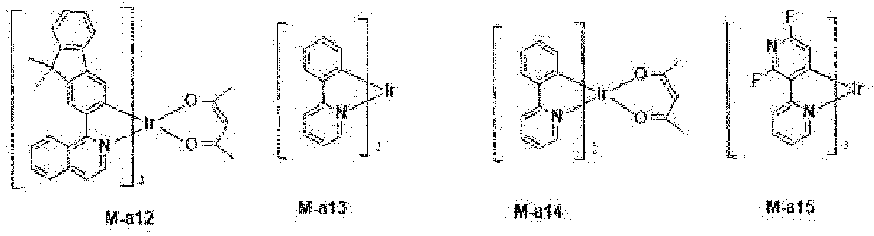

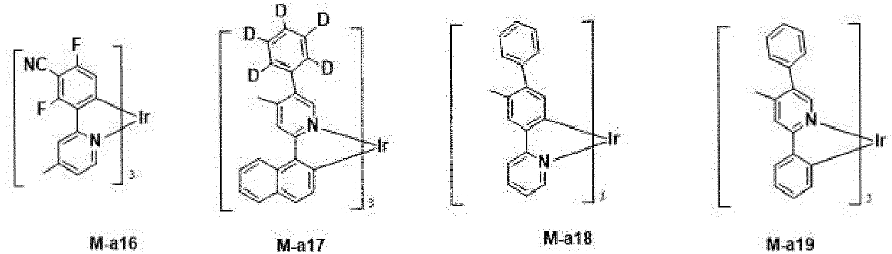

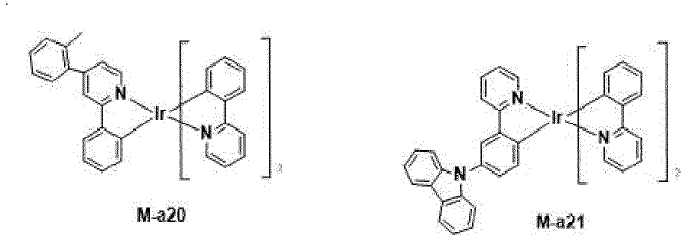

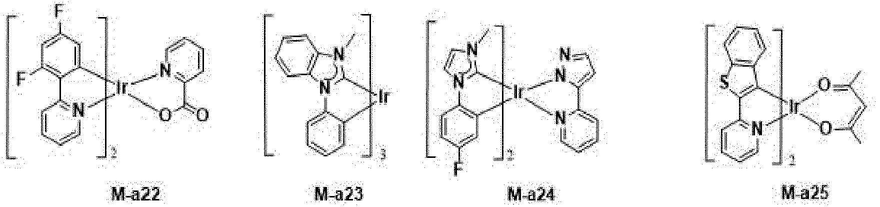

The compounds M-a1 and M-a2 may be utilized as a red dopant material, and the compounds M-a3 and M-a4 may be utilized as a green dopant material.

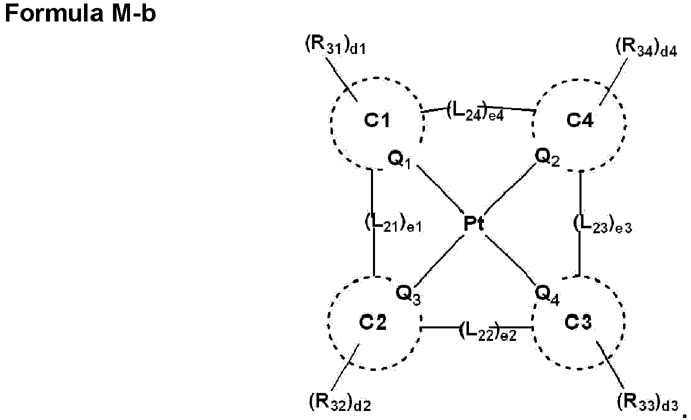

In Formula M-b, Q₁ to Q4 are each independently C or N, and C1 to C4 are each independently a substituted or unsubstituted hydrocarbon ring having 5 to 30 ring-forming carbon atoms, or a substituted or unsubstituted heterocycle having 2 to 30 ring-forming carbon atoms. L₂₁ to L₂₄ are each independently a direct linkage, 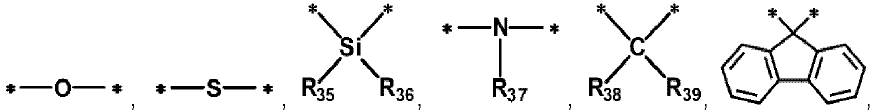 a substituted or unsubstituted divalent alkyl group having 1 to 20 carbon atoms, a substituted or unsubstituted arylene group having 6 to 30 ring-forming carbon atoms, or a substituted or unsubstituted heteroarylene group having 2 to 30 ring-forming carbon atoms, and e1 to e4 are each independently 0 or 1. R₃₁ to R₃₉ are each independently a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring, and d1 to d4 are each independently an integer of 0 to 4.

The compound represented by Formula M-b may be utilized as a blue phosphorescent dopant or a green phosphorescent dopant.

The compound represented by Formula M-b may be represented by any one of the compounds below. However, the compounds below are presented as an example, and the compound represented by Formula M-b is not limited to those represented by the compounds below.

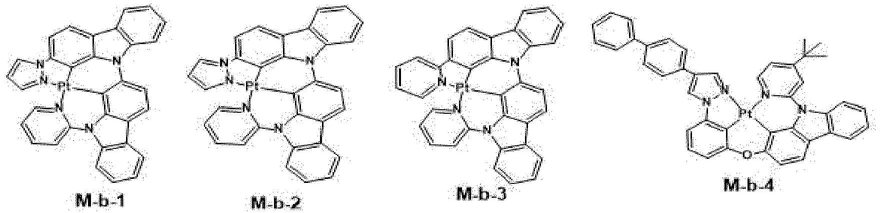

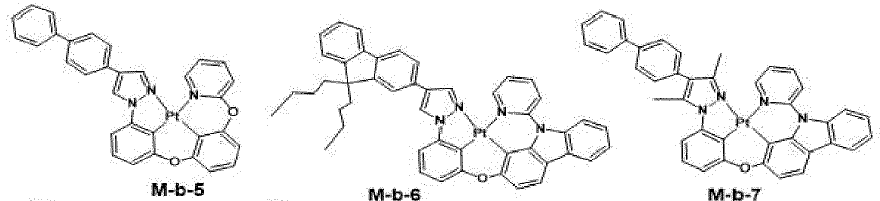

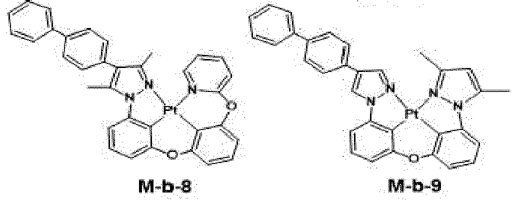

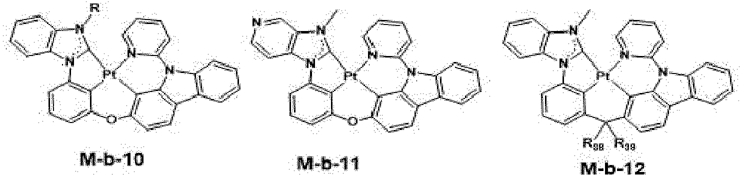

In the compounds above, R, R₃₈, and R₃₉ are each independently a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

The emission layer EML may include a compound represented by any one of Formulae F-a to F-c below. The compounds represented by Formulae F-a to F-c below may be utilized as a fluorescent dopant material.

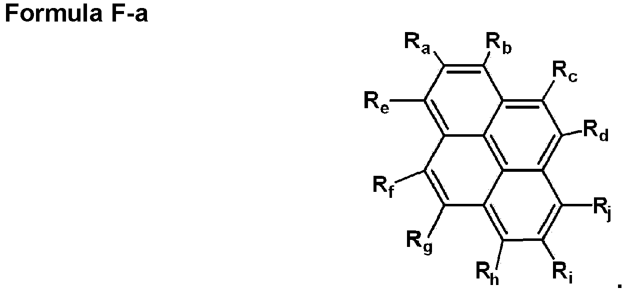

In Formula F-a above, two selected from Rₐ to Rⱼ are each independently represented by (e.g., substituted with) ∗-NAr₁Ar₂. The rest of Rₐ to Rⱼ which are not substituted with ∗-NAr₁Ar₂ are each independently a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. In ∗-NAr₁Ar₂, Ar₁ and Ar₂ are each independently a substituted or unsubstituted aryl group having 6 to 30 ring-forming carbon atoms, or a substituted or unsubstituted heteroaryl group having 2 to 30 ring-forming carbon atoms. For example, at least one of Ar₁ or Ar₂ may be a heteroaryl group containing O or S as a ring-forming atom.

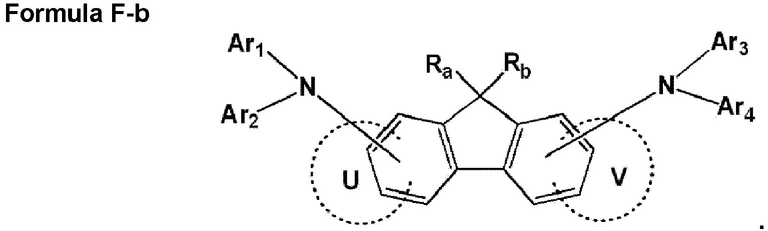

In Formula F-b above, Rₐ and R_{b} are each independently a hydrogen atom, a deuterium atom, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, a substituted or unsubstituted heteroaryl group having 2 to 30 ring-forming carbon atoms; and/or bonded to an adjacent group to form a ring. Ar₁ to Ar₄ may be each independently a substituted or unsubstituted aryl group of 6 to 30 ring-forming carbon atoms, or a substituted or unsubstituted heteroaryl group of 2 to 30 ring-forming carbon atoms, or may be combined with an adjacent group to form a ring.

In Formula F-b, U and V are each independently a substituted or unsubstituted hydrocarbon ring having 5 to 30 ring-forming carbon atoms, or a substituted or unsubstituted heterocycle having 2 to 30 ring-forming carbon atoms.

In Formula F-b, the number of rings represented by U and V may be each independently 0 or 1. For example, In Formula F-b, when the number of U or V is 1, one ring forms a condensed ring in a portion (e.g., position) indicated by U or V, and when the number of U or V is 0, it indicates that no ring in the position indicated by U or V is present. For example, when the number of U is 0 and the number of V is 1, or when the number of U is 1 and the number of V is 0, a condensed ring having a fluorene core of Formula F-b may be a cyclic compound having four rings. In addition, when both U and V are 0, the condensed ring of Formula F-b may be a cyclic compound having three rings. In addition, when both U and V are 1, the condensed ring having a fluorene core of Formula F-b may be a cyclic compound having five rings.

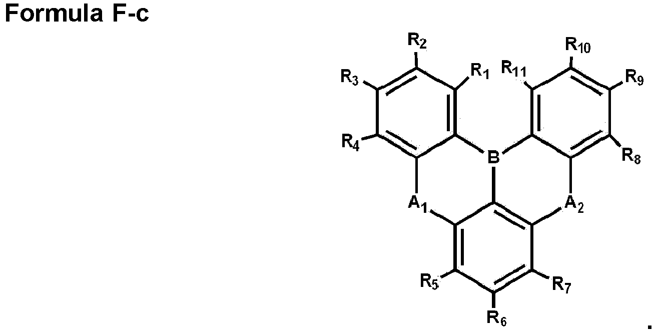

In Formula F-c, A₁ and A₂ are each independently O, S, Se, or NRₘ, and Rₘ is a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. R₁ to R₁₁ are each independently a hydrogen atom, a deuterium atom, a halogen atom, a cyano group, a substituted or unsubstituted amine group, a substituted or unsubstituted boryl group, a substituted or unsubstituted oxy group, a substituted or unsubstituted thio group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring.

In Formula F-c, A₁ and A₂ may be each independently bonded to substituents of neighboring rings to form a condensed ring. For example, when A₁ and A₂ are each independently NRₘ, A₁ may be bonded to R₄ or R₅ to form a ring. In addition, A₂ may be bonded to R₇ or R₈ to form a ring.

The emission layer EML may include, as a suitable (e.g., known) dopant material, styryl derivatives (e.g., 1,4-bis[2-(3-N-ethylcarbazoryl)vinyl]benzene (BCzVB), 4-(di-p-tolylamino)-4"-[(di-p-tolylamino)styryl]stilbene (DPAVB), and/or N-(4-((E)-2-(6-((E)-4-(diphenylamino)styryl)naphthalen-2-yl)vinyl)phenyl)-N-phenylbenzenamine (N-BDAVBi)), perylene and derivatives thereof (e.g., 2,5,8,11-tetra-t-butylperylene (TBP)), pyrene and derivatives thereof (e.g., 1,1'-dipyrene, 1,4-dipyrenylbenzene, and/or 1,4-bis(N,N-diphenylamino)pyrene), etc.

The emission layer EML may include a suitable (e.g., known) phosphorescent dopant material. For example, as a phosphorescent dopant, a metal complex including iridium (Ir), platinum (Pt), osmium (Os), gold (Au), titanium (Ti), zirconium (Zr), hafnium (Hf), europium (Eu), terbium (Tb), and/or thulium (Tm) may be utilized. For example, iridium(III) bis(4,6-difluorophenylpyridinato-N,C2')picolinate (Flrpic), bis(2,4-difluorophenylpyridinato)-tetrakis(1-pyrazolyl)borate iridium(III) (Fir6), platinum octaethyl porphyrin (PtOEP), etc. may be utilized as a phosphorescent dopant. However, the embodiment of the present disclosure is not limited thereto.

The emission layer EML may include a quantum dot material. The quantum dot material (e.g., the core of a quantum dot) may be selected from a Group II-VI compound, a Group III-VI compound, a Group I-III-VI compound, a Group III-V compound, a Group III-II-V compound, a Group IV-VI compound, a Group IV element, a Group IV compound, and a combination thereof.

The Group II-VI compound may be selected from the group consisting of a binary compound selected from the group consisting of CdSe, CdTe, CdS, ZnS, ZnSe, ZnTe, ZnO, HgS, HgSe, HgTe, MgSe, MgS, and a mixture thereof, a ternary compound selected from the group consisting of CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and a mixture thereof, and a quaternary compound selected from the group consisting of HgZnTeS, CdZnSeS, CdZnSeTe, CdZnSTe, CdHgSeS, CdHgSeTe, CdHgSTe, HgZnSeS, HgZnSeTe, HgZnSTe, and a mixture thereof.

The Group III-VI compound may include a binary compound such as In₂S₃ and/or In₂Se₃, a ternary compound such as InGaS₃ and/or InGaSe₃, or any combination thereof.

The Group I-III-VI compound may include a ternary compound selected from the group consisting of AglnS, AgInS₂, CulnS, CuInS₂, AgGaS₂, CuGaS₂ CuGaO₂, AgGaO₂, AgAlO₂, or any mixture thereof, and/or a quaternary compound such as AgInGaS₂ and/or CuInGaS₂.

The Group III-V compound may be selected from the group consisting of a binary compound selected from the group consisting of GaN, GaP, GaAs, GaSb, AIN, AIP, AlAs, AlSb, InN, InP, InAs, InSb, and a mixture thereof, a ternary compound selected from the group consisting of GaNP, GaNAs, GaNSb, GaPAs, GaPSb, AINP, AINAs, AINSb, AlPAs, AlPSb, InGaP, InAlP, InNP, InNAs, InNSb, InPAs, InPSb, and a mixture thereof, and a quaternary compound selected from the group consisting of GaAINP, GaAINAs, GaAINSb, GaAlPAs, GaAlPSb, GaInNP, GaInNAs, GaInNSb, GaInPAs, GaInPSb, InAlNP, InAlNAs, InAlNSb, InAlPAs, InAlPSb, and a mixture thereof. In some embodiments, the Group III-V compound may further include a Group II metal. For example, InZnP, etc. may be selected as a Group III-II-V compound.

The Group IV-VI compound may be selected from the group consisting of a binary compound selected from the group consisting of SnS, SnSe, SnTe, PbS, PbSe, PbTe, and a mixture thereof, a ternary compound selected from the group consisting of SnSeS, SnSeTe, SnSTe, PbSeS, PbSeTe, PbSTe, SnPbS, SnPbSe, SnPbTe, and a mixture thereof, and a quaternary compound selected from the group consisting of SnPbSSe, SnPbSeTe, SnPbSTe, and a mixture thereof. The Group IV element may be selected from the group consisting of Si, Ge, and a mixture thereof. The Group IV compound may be a binary compound selected from the group consisting of SiC, SiGe, and a mixture thereof.

In this case, the binary compound, the ternary compound, and/or the quaternary compound may be present in particles in a uniform concentration distribution, or may be present in the same particles in a partially different concentration distribution. In addition, a core/shell structure in which one quantum dot surrounds another quantum dot may be present. The core/shell structure may have a concentration gradient in which the concentration of an element present in the shell becomes lower towards the center of the core.

In some embodiments, a quantum dot may have the core/shell structure including a core including nano-crystals, and a shell around (e.g., surrounding) the core, which are described above. The shell of the quantum dot may serve as a protection layer to prevent or substantially prevent the chemical deformation of the core so as to keep semiconductor properties, and/or as a charging layer to impart electrophoresis properties to the quantum dot. The shell may be a single layer or multiple layers. Examples of the shell of the quantum dot may include (e.g., may be) a metal or non-metal oxide, a semiconductor compound, or a combination thereof.

For example, the metal or non-metal oxide may be a binary compound such as SiO₂, Al₂O₃, TiO₂, ZnO, MnO, Mn₂O₃, Mn₃O₄, CuO, FeO, Fe₂O₃, Fe₃O₄, CoO, Co₃O₄, and/or NiO, and/or a ternary compound such as MgAl₂O₄, CoFe₂O₄, NiFe₂O₄, and/or CoMn₂O₄, but the embodiment of the present disclosure is not limited thereto.

In addition, the semiconductor compound may be, for example, CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, etc., but the embodiment of the present disclosure is not limited thereto.

A quantum dot may have a full width of half maximum (FWHM) of a light emitting wavelength spectrum of about 45 nm or less, for example, about 40 nm or less, or about 30 nm or less, and color purity or color reproducibility may be enhanced in the above ranges. In addition, light emitted through such a quantum dot is emitted in all directions, and thus a wide viewing angle may be improved.

In addition, the form of a quantum dot is not particularly limited as long as it is a form commonly utilized in the art, and for example, a quantum dot in the form of spherical, pyramidal, multi-arm, or cubic nanoparticles, nanotubes, nanowires, nanofibers, nanoplatelets, etc. may be utilized.

The quantum dot may control the color of emitted light according to particle size thereof, and thus the quantum dot may have various suitable colors of emitted light such as blue, red, green, etc.

In the luminescence device ED of an embodiment shown in FIGS. 3 to 6, an electron transport region ETR is provided on the emission layer EML. The electron transport region ETR may include at least one among a hole blocking layer HBL, an electron transport layer ETL, and an electron injection layer EIL, but the embodiment of the present disclosure is not limited thereto.

The electron transport region ETR may have a single layer formed of a single material, a single layer formed of a plurality of different materials, or a multilayer structure having a plurality of layers formed of a plurality of different materials.

For example, the electron transport region ETR may have a single layer structure of an electron injection layer EIL or an electron transport layer ETL, and may have a single layer structure formed of an electron injection material and an electron transport material. In addition, the electron transport region ETR may have a single layer structure formed of a plurality of different materials, or may have a structure in which an electron transport layer ETL/electron injection layer EIL, or a hole blocking layer HBL/electron transport layer ETL/electron injection layer EIL are stacked in the respective stated order from the emission layer EML, but the present disclosure is not limited thereto. The electron transport region ETR may have a thickness of, for example, about 1000 Å to about 1500 Å.

The electron transport region ETR may be formed utilizing various suitable methods such as a vacuum deposition method, a spin coating method, a cast method, a Langmuir-Blodgett (LB) method, an inkjet printing method, a laser printing method, a laser induced thermal imaging (LITI) method, etc.

In an embodiment, the electron transport region ETR may include a compound represented by Formula ET-1 below.

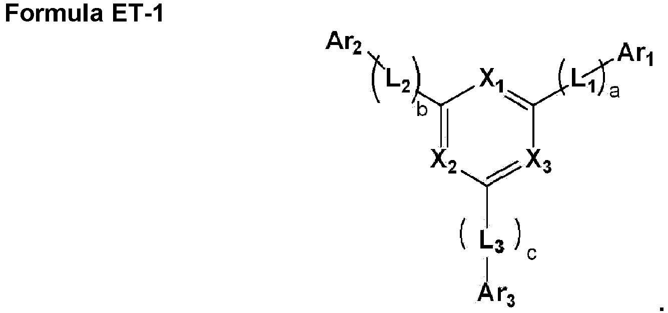

In Formula ET-1, at least one of X₁ to X₃ is N and the rest (e.g., a remainder thereof) are each independently CRₐ. Rₐ is a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. Ar₁ to Ar₃ are each independently a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In Formula ET-1, a to c are each independently an integer of 0 to 10. In Formula ET-1, L₁ to L₃ are each independently a direct linkage, a substituted or unsubstituted arylene group having 6 to 30 ring-forming carbon atoms, or a substituted or unsubstituted heteroarylene group having 2 to 30 ring-forming carbon atoms. In some embodiments, when a to c are an integer of 2 or greater, L₁ to L₃ may be each independently a substituted or unsubstituted arylene group having 6 to 30 ring-forming carbon atoms, or a substituted or unsubstituted heteroarylene group having 2 to 30 ring-forming carbon atoms.

The electron transport region ETR may include an anthracene-based compound. However, the embodiment of the present disclosure is not limited thereto, and the electron transport region ETR may include, for example, tris(8-hydroxyquinolinato)aluminium (Alq₃), 1,3,5-tri[(3-pyridyl)-phen-3-yl]benzene, 2,4,6-tris(3'-(pyridin-3-yl)biphenyl-3-yl)-1 ,3,5-triazine, 2-(4-(N-phenylbenzoimidazolyl-1 - yl)phenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,O8)-(1,1'-biphenyl-4-olato)aluminium (BAIq), berylliumbis(benzoquinolin-10-olate) (Bebq₂), 9,10-di(naphthalene-2-yl)anthracene (ADN), 1,3-bis[3,5-di(pyridin-3-yl)phenyl]benzene (BmPyPhB), or a mixture thereof.

The electron transport region ETR may include at least one of compounds ET1 to ET36 below.

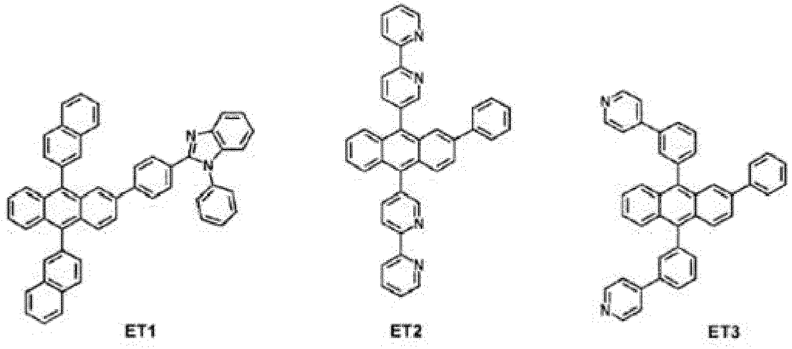

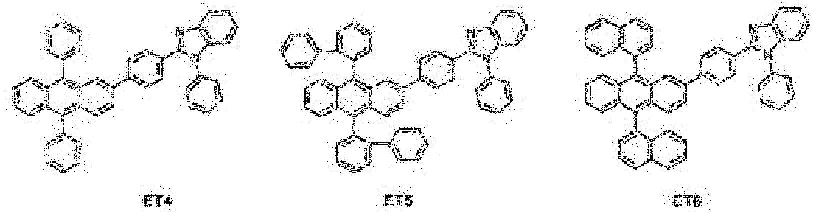

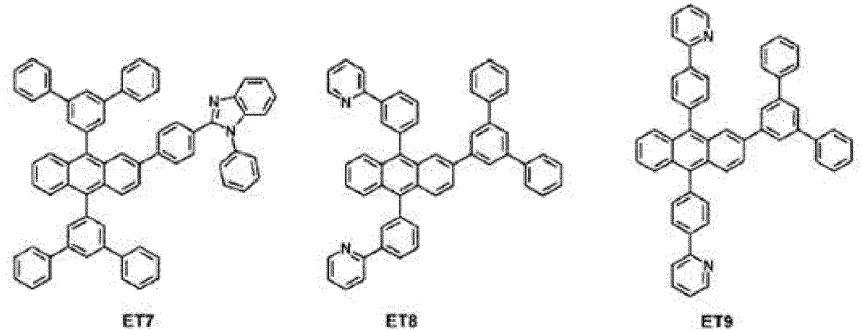

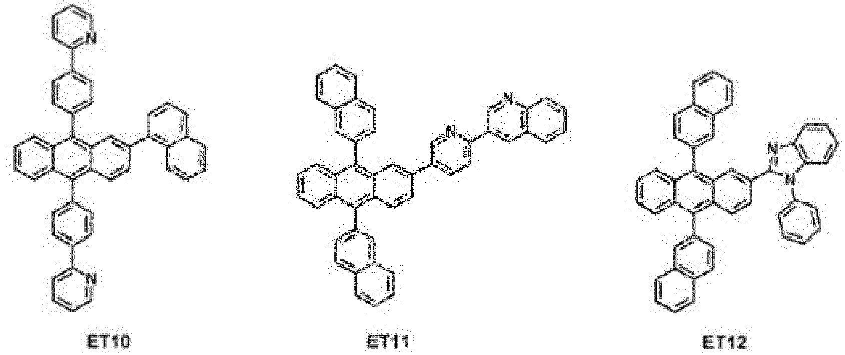

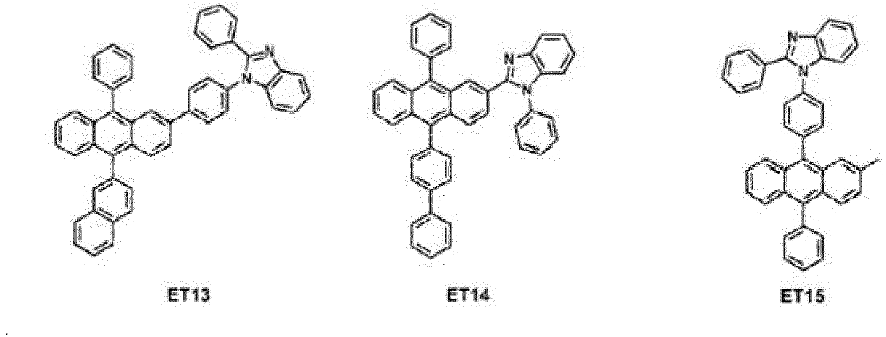

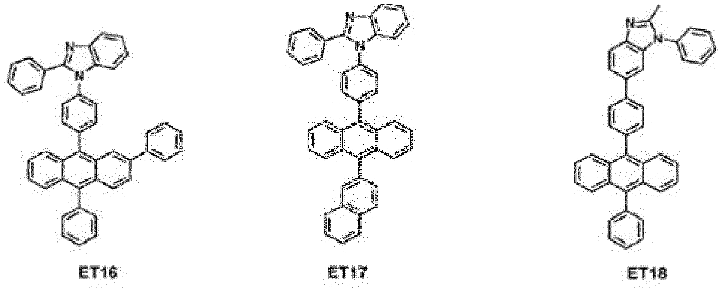

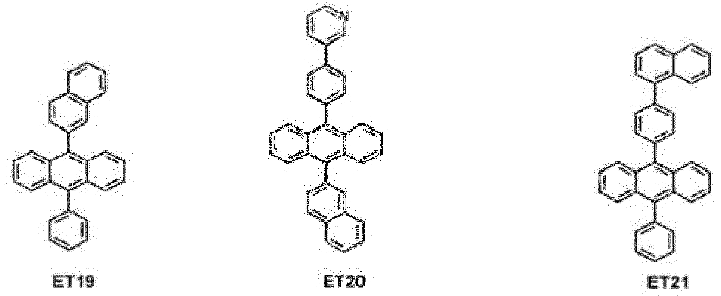

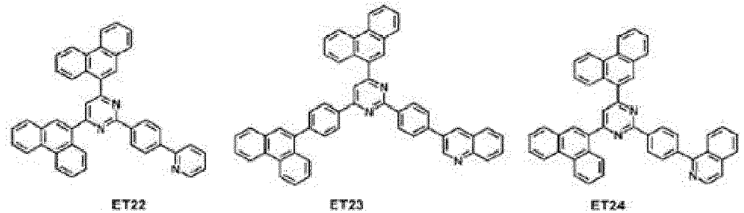

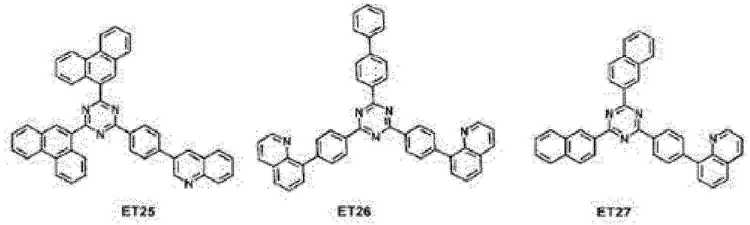

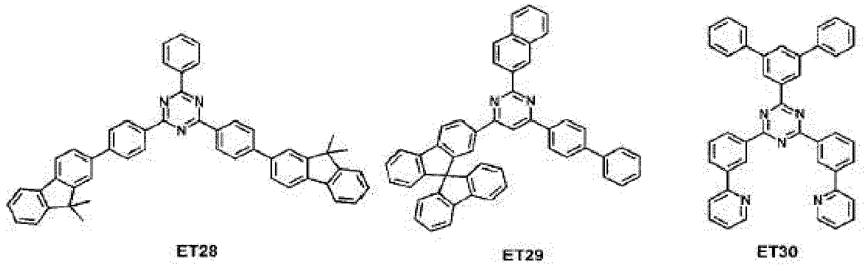

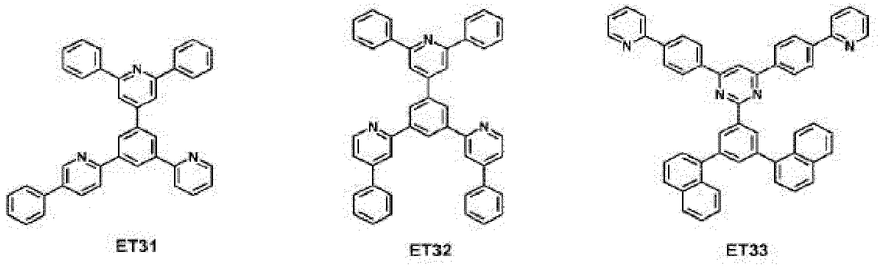

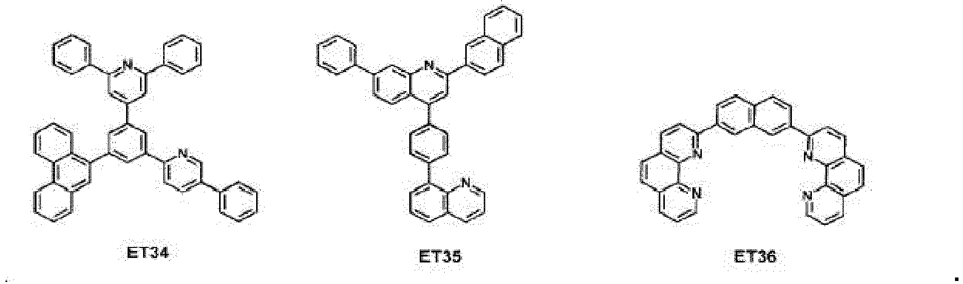

In addition, the electron transport region ETR may include one or more halogenated metals such as LiF, NaCl, CsF, RbCl, RbI, CuI, and/or KI, one or more lanthanide metals such as Yb, one or more co-deposition materials of a halogenated metal and a lanthanide metal. For example, the electron transport region ETR may include KI:Yb, RbI:Yb, etc. as a co-deposition material. In some embodiments, for the electron transport region ETR, a metal oxide such as Li₂O and/or BaO, or 8-hydroxyl-lithium quinolate (Liq), etc. may be utilized, but the embodiment of the present disclosure is limited thereto. The electron transport region ETR may also be formed of a mixture material of an electron transport material and an insulating organo-metal salt. The organo-metal salt may be a material having an energy band gap of about 4 eV or greater. For example, the organo-metal salt may include, for example, one or more metal acetates, metal benzoates, metal acetoacetates, metal acetylacetonates, and/or metal stearates.

The electron transport region ETR may further include, for example, at least one of 2,9-dimethyl-4,7-diphenyl-1,10-phenanthroline (BCP) or 4,7-diphenyl-1,10-phenanthroline (Bphen) in addition to the materials described above, but the embodiment of the present disclosure is not limited thereto.

The electron transport region ETR may include the compounds of the electron transport region ETR described above in at least one among the electron injection layer EIL, the electron transport layer ETL, and the hole blocking layer HBL.

When the electron transport region ETR includes the electron transport layer ETL, the electron transport layer ETL may have a thickness of about 100 Å to about 1000 Å, for example, about 150 Å to about 500 Å. When the thickness of the electron transport layer ETL satisfies the above-described ranges, satisfactory electron transport properties may be obtained without a substantial increase in driving voltage. When the electron transport region ETR includes the electron injection layer EIL, the electron injection layer EIL may have a thickness of about 1 Å to about 100 Å, for example, about 3 Å to about 90 Å. When the thickness of the electron injection layer EIL satisfies the above-described ranges, satisfactory electron injection properties may be obtained without a substantial increase in driving voltage.

The second electrode EL2 is provided on the electron transport region ETR. The second electrode EL2 may be a common electrode. The second electrode EL2 may be a cathode or an anode, but the embodiment of the present disclosure is not limited thereto. For example, when the first electrode EL1 is an anode, the second electrode EL2 may be a cathode, and when the first electrode EL1 is a cathode, the second electrode EL2 may be an anode.

The second electrode EL2 may be a transmissive electrode, a transflective electrode, or a reflective electrode. When the second electrode EL2 is a transmissive electrode, the second electrode EL2 may be formed of a transparent metal oxide, for example, indium tin oxide (ITO), indium zinc oxide (IZO), zinc oxide (ZnO), indium tin zinc oxide (ITZO), etc.

When the second electrode EL2 is a transflective electrode or a reflective electrode, the second electrode EL2 may include Ag, Mg, Cu, Al, Pt, Pd, Au, Ni, Nd, Ir, Cr, Li, Ca, LiF/Ca, LiF/Al, Mo, Ti, Yb, W, a compound thereof, or a mixture thereof (e.g., AgMg, AgYb, or MgAg). In some embodiments, the second electrode EL2 may have a multilayer structure including a reflective film or a transflective film formed of the above-described materials, and a transparent conductive film formed of indium tin oxide (ITO), indium zinc oxide (IZO), zinc oxide (ZnO), indium tin zinc oxide (ITZO), etc. For example, the second electrode EL2 may include the above-described metal materials, a combination of two or more metal materials selected from the above-described metal materials, and/or oxides of the above-described metal materials.

In some embodiments, the second electrode EL2 may be connected with an auxiliary electrode. When the second electrode EL2 is connected with the auxiliary electrode, the resistance of the second electrode EL2 may decrease.

In some embodiments, a capping layer CPL may be further disposed on the second electrode EL2 of the luminescence device ED of an embodiment. The capping layer CPL may include a multilayer or a single layer.

In an embodiment, the capping layer CPL may be an organic layer or an inorganic layer. For example, when the capping layer CPL includes an inorganic material, the inorganic material may include an alkali metal compound such as LiF, an alkaline earth metal compound such as MgF₂, SiON, SiNₓ, SiO_{y}, etc.

For example, when the capping layer CPL includes an organic material, the organic material may include α-NPD, NPB, TPD, m-MTDATA, Alq₃ CuPc, N4,N4,N4',N4'-tetra(biphenyl-4-yl) biphenyl-4,4'-diamine (TPD15), 4,4',4"-tris(carbazol-9-yl)triphenylamine (TCTA), etc., or may include epoxy resins or acrylates such as methacrylates. However, the embodiment of the present disclosure is not limited thereto, and the capping layer CPL may further include one or more of compounds P1 to P5 below.

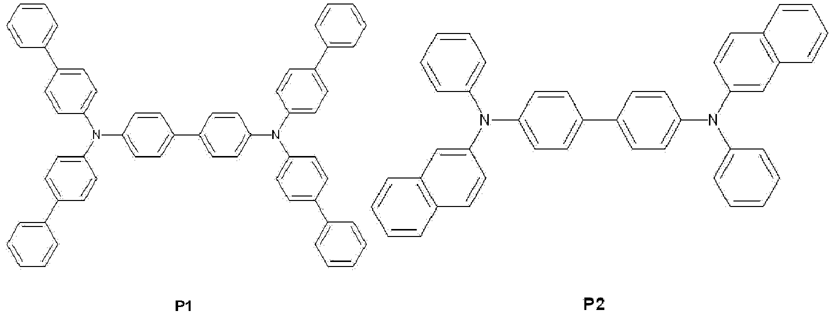

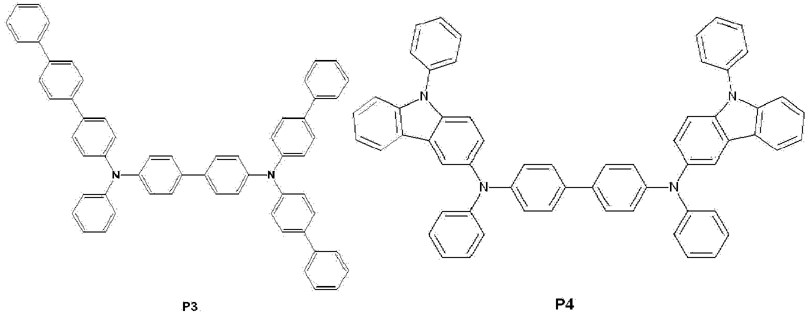

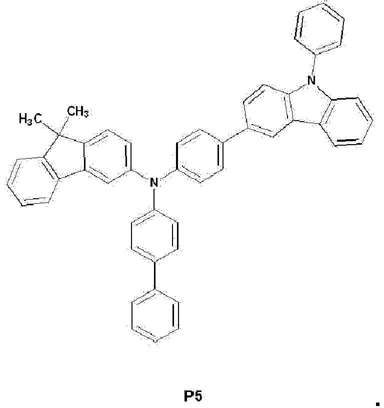

In some embodiments, the capping layer CPL may have a refractive index of about 1.6 or greater. For example, the capping layer CPL may have a refractive index of about 1.6 or greater in a wavelength range of about 550 nm to about 660 nm.

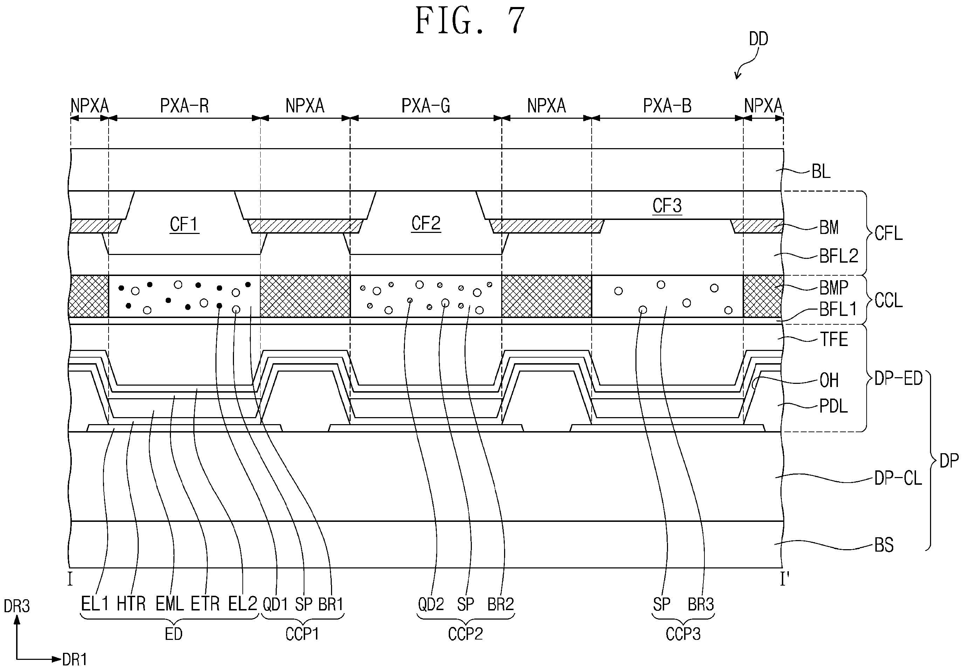

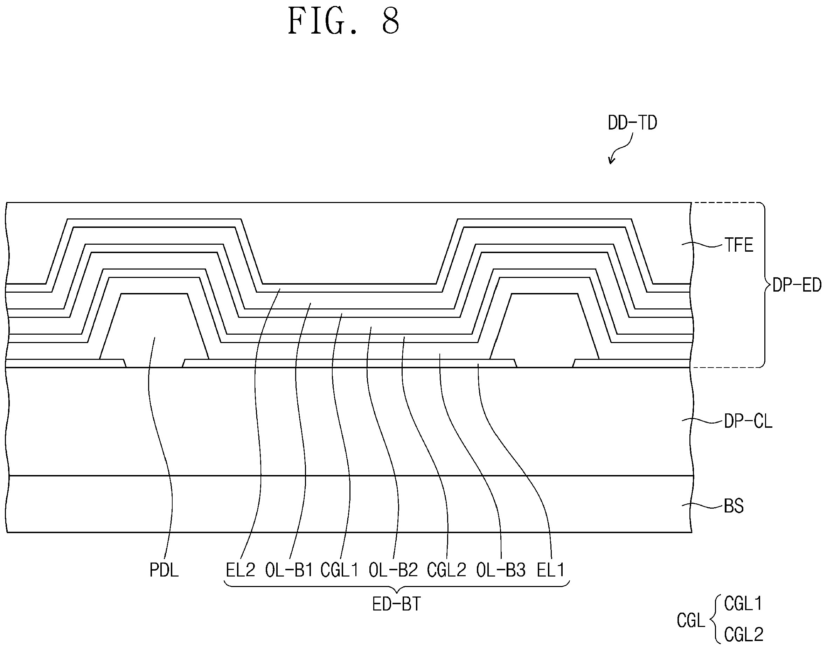

FIGS. 7 and 8 each are cross-sectional views of a display apparatus according to an embodiment. Hereinafter, in the description of the display apparatus according to an embodiment with reference to FIGS. 7 and 8, content overlapping the one described above with reference to FIGS. 1 to 6 will not be described again, and the differences will be mainly described.

Referring to FIG. 7, a display apparatus DD according to an embodiment may include a display panel DP having a display element layer DP-ED, a light control layer CCL disposed on the display panel DP, and a color filter layer CFL.

In an embodiment shown in FIG. 7, the display panel DP may include a base layer BS, a circuit layer DP-CL provided on the base layer BS, and a display element layer DP-ED, and the element layer DP-ED may include a luminescence device ED.

The luminescence device ED may include a first electrode EL1, a hole transport region HTR disposed on the first electrode EL1, an emission layer EML disposed on the hole transport region HTR, an electron transport region ETR disposed on the emission layer EML, and a second electrode EL2 disposed on the electron transport region ETR. In some embodiments, a structure of the luminescence device ED shown in FIG. 7 may be the same as the structure of the luminescence deviceof FIGS. 3 to 6 described above.

Referring to FIG. 7, the emission layer EML may be disposed in the openings OH defined in the pixel defining films PDL. For example, the emission layer EML separated by the pixel defining films PDL and provided corresponding to each of light emitting areas PXA-R, PXA-G, and PXA-B may emit light in the same wavelength ranges. In the display apparatus DD of an embodiment, the emission layer EML may emit blue light. In some embodiments, unlike what is shown, in an embodiment, the emission layer EML may be provided as a common layer throughout the light emitting areas PXA-R, PXA-G, and PXA-B.

The light control layer CCL may be disposed on the display panel DP. The light control layer CCL may include a photoconverter. The photoconverter may be a quantum dot and/or a phosphor. The photoconverter may convert the wavelength of received light, and emit the resulting light. That is, the light control layer CCL may be a layer containing quantum dots and/or phosphors.

The light control layer CCL may include a plurality of light control units CCP1, CCP2, and CCP3. The light control units CCP1, CCP2, and CCP3 may be spaced apart from each other.

Referring to FIG. 7, a division pattern BMP may be disposed between the light control units CCP1, CCP2, and CCP3 spaced apart from each other, but the embodiment of the present disclosure is not limited thereto. In FIG. 7, the division pattern BMP is shown to not overlap the light control units CCP1, CCP2, and CCP3, but in some embodiments, edges of the light control units CCP1, CCP2, and CCP3 may overlap at least a portion of the division pattern BMP.

The light control layer CCL may include a first light control unit CCP1 including a first quantum dot QD1 for converting a first color light provided from the luminescence device ED into a second color light, a second light control unit CCP2 including a second quantum dot QD2 for converting the first color light into a third color light, and a third light control unit CCP3 transmitting the first color light.

In an embodiment, the first light control unit CCP1 may provide red light, which is the second color light, and the second light control unit CCP2 may provide green light, which is the third color light. The third light control unit CCP3 may transmit and provide blue light, which is the first color light provided from the luminescence device ED. For example, the first quantum dot QD1 may be a red quantum dot (e.g., red-light emitting quantum dot) and the second quantum dot QD2 may be a green quantum dot (e.g., green-light emitting quantum dot). The same descriptions above may be applied to the quantum dots QD1 and QD2.

In addition, the light control layer CCL may further include scatterers SP. The first light control unit CCP1 may include the first quantum dot QD1 and the scatterers SP, the second light control unit CCP2 may include the second quantum dot QD2 and the scatterers SP, and the third light control unit CCP3 may not include a quantum dot but may include the scatterers SP.

The scatterers SP may be inorganic particles. For example, the scatterers SP may include at least one among TiO₂, ZnO, Al₂O₃, SiO₂, and hollow silica. The scatterers SP may include any one among TiO₂, ZnO, Al₂O₃, SiO₂, and hollow silica, or may be a mixture of two or more materials selected from TiO₂, ZnO, Al₂O₃, SiO₂, and hollow silica.

The first light control unit CCP1, the second light control unit CCP2, and the third light control unit CCP3 may each include base resins BR1, BR2, and BR3 respectively for dispersing the quantum dots QD1 and QD2 and the scatterers SP. In an embodiment, the first light control unit CCP1 may include the first quantum dot QD1 and the scatterers SP dispersed in the first base resin BR1, the second light control unit CCP2 may include the second quantum dot QD2 and the scatterers SP dispersed in the second base resin BR2, and the third light control unit CCP3 may include the scatterers SP dispersed in the third base resin BR3. The base resins BR1, BR2, and BR3 are a medium in which the quantum dots QD1 and QD2 and the scatterers SP are respectively dispersed, and may be formed of various suitable resin compositions, which may be generally referred to as a binder. For example, the base resins BR1, BR2, and BR3 may be an acrylic (e.g., acrylic-based) resin, a urethane-based resin, a silicone-based resin, an epoxy-based resin, etc. The base resins BR1, BR2, and BR3 may be a transparent resin. In an embodiment, the first base resin BR1, the second base resin BR2, and the third base resin BR3 may each be the same as or different from each other.

The light control layer CCL may include a barrier layer BFL1. The barrier layer BFL1 may serve to prevent or substantially prevent moisture and/or oxygen (hereinafter referred to as "moisture/oxygen") from being introduced. The barrier layer BFL1 may be disposed on the light control units CCP1, CCP2, and CCP3 to prevent or substantially prevent the light control units CCP1, CCP2, and CCP3 from being exposed to moisture/oxygen. In some embodiments, the barrier layer BFL1 may cover the light control units CCP1, CCP2, and CCP3. In addition, a barrier layer BFL2 may be provided between the light control units CCP1, CCP2, and CCP3 and the color filter layer CFL.

The barrier layers BFL1 and BFL2 may include at least one inorganic layer. That is, the barrier layers BFL1 and BFL2 may be formed of an inorganic material. For example, the barrier layers BFL1 and BFL2 may be formed to include silicon nitride, aluminium nitride, zirconium nitride, titanium nitride, hafnium nitride, tantalum nitride, silicon oxide, aluminium oxide, titanium oxide, tin oxide, cerium oxide, silicon oxynitride, and/or a metal thin film in which light transmittance is secured, etc. In some embodiments, the barrier layers BFL1 and BFL2 may further include an organic film. The barrier layers BFL1 and BFL2 may be formed of a single layer or a plurality of layers.

In the display apparatus DD of an embodiment, the color filter layer CFL may be disposed on the light control layer CCL. For example, the color filter layer CFL may be directly disposed on the light control layer CCL. In this case, the barrier layer BFL2 may be omitted.

The color filter layer CFL may include a light blocking unit BM and filters CF1, CF2, and CF3. That is, the color filter layer CFL may include a first filter CF1 transmitting the second color light, a second filter CF2 transmitting the third color light, and a third filter CF3 transmitting the first color light. For example, the first filter CF1 may be a red filter, the second filter CF2 may be a green filter, and the third filter CF3 may be a blue filter. The filters CF1, CF2, and CF3 may each include a polymer photosensitive resin, a pigment and/or a dye. The first filter CF1 may include a red pigment and/or a red dye, the second filter CF2 may include a green pigment and/or a green dye, and the third filter CF3 may include a blue pigment and/or a blue dye. In some embodiments, the embodiment of the present disclosure is not limited thereto, and the third filter CF3 may not include a pigment and/or a dye. The third filter CF3 may include a polymer photosensitive resin, but not include a pigment and/or a dye. The third filter CF3 may be transparent. The third filter CF3 may be formed of a transparent photosensitive resin.

In addition, in an embodiment, the first filter CF1 and the second filter CF2 may be yellow filters. The first filter CF1 and the second filter CF2 may not be separated from each other and may be provided as a single body.

The light blocking unit BM may be a black matrix. The light blocking unit BM may be formed including an organic light blocking material or an inorganic light blocking material, both including a black pigment or a black dye. The light blocking unit BM may prevent or reduce light leakage, and separate boundaries between the adjacent filters CF1, CF2, and CF3. In addition, in an embodiment, the light blocking unit BM may be formed of a blue filter.

The first to third filters CF1, CF2, and CF3 may be disposed corresponding to the red light emitting area PXA-R, green light emitting area PXA-G, and blue light emitting area PXA-B, respectively.

The base substrate BL may be disposed on the color filter layer CFL. The base substrate BL may be a member providing a base surface on which the color filter layer CFL and the light control layer CCL are disposed. The base substrate BL may be a glass substrate, a metal substrate, a plastic substrate, etc. However, the embodiment of the present disclosure is not limited thereto, and the base substrate BL may be an inorganic layer, an organic layer, or a composite material layer. In addition, unlike what is shown, the base substrate BL may be omitted in an embodiment.

FIG. 8 is a cross-sectional view showing a portion of a display apparatus according to an embodiment. FIG. 8 shows a cross-sectional view of a portion corresponding to the display panel DP of FIG. 7. In a display apparatus DD-TD of an embodiment, a luminescence device ED-BT may include a plurality of light emitting structures OL-B1, OL-B2, and OL-B3. The luminescence device ED-BT may include the first electrode EL1 and the second electrode EL2 facing each other, and the plurality of light emitting structures OL-B1, OL-B2, and OL-B3 provided by being sequentially stacked in a thickness direction between the first electrode EL1 and the second electrode EL2. The light emitting structures OL-B1, OL-B2, and OL-B3 each may include the emission layer EML (FIG. 7), and a hole transport region HTR and an electron transport region ETR disposed with the emission layer EML (FIG. 7) therebetween.

That is, the luminescence device ED-BT included in the display apparatus DD-TD of an embodiment may be a luminescence device having a tandem structure including a plurality of emission layers.

In an embodiment shown in FIG. 8, light emitted from each of the light emitting structures OL-B1, OL-B2, and OL-B3 may all be blue light. However, the embodiment of the present disclosure is not limited thereto, and wavelength ranges of light emitted from each of the light emitting structures OL-B1, OL-B2, and OL-B3 may be different from each other. For example, the luminescence device ED-BT including the plurality of light emitting structures OL-B1, OL-B2, and OL-B3 emitting light in different wavelength ranges may emit white light.

A charge generation layer CGL (CGL1 and CGL2) may be disposed between neighboring light emitting structures OL-B1, OL-B2, and OL-B3. The charge generation layer CGL may include a p-type charge generation layer and/or an n-type charge generation layer.

Hereinafter, embodiments of the present disclosure will be described in more detail through specific Examples and Comparative Examples. Examples shown below are illustrated only for the understanding of the present disclosure, and the scope of the present disclosure is not limited thereto.

### Synthesis Examples

Nitrogen-containing compounds according to an embodiment of the present disclosure may be synthesized, for example, as follows. However, a process of the synthesizing of nitrogen-containing compounds according to an embodiment of the present disclosure is not limited to thereto.

### 1. Synthesis of Compound 1

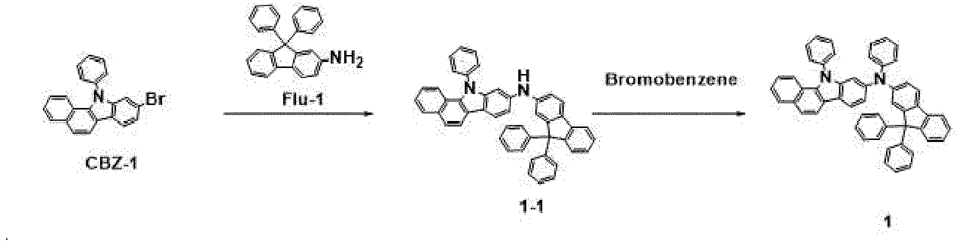

### (1) Synthesis of Intermediate 1-1

CBZ-1 (7.420 g, 20 mmol), Flu-1 (6.662 g, 20 mmol), Pd₂(dba)₃ (0.915 g, 1 mol), (t-Bu)₃P (0.096 g, 1 mol), and NaOtBu (3.6 g, 40 mmol) were dissolved in toluene (200 ml) and stirred at 90 °C for 2 hours, and extracted 3 times with Et₂O/H₂O. The resultant was dried with anhydrous magnesium sulfate and then separated and purified utilizing column chromatography, thereby obtaining Intermediate 1-1 (11.23 g, 18 mmol), with a yield of 90%.

### (2) Synthesis of Compound 1

Compound 1 (5.6 g, 9 mmol), with a yield of 90%, was obtained in the same manner as in the synthesis of Intermediate 1-1, except that Intermediate 1-1 was utilized instead of CBZ-1, and bromobenzene was utilized instead of Flu-1. (C₅₃H₃₆N₂ M+1: 700.29, ¹H NMR (500 MHz, CDCl₃) δ= 7.80 (m, 2H), 7.60 (d, 1H), 7.55-7.10(m, 22H), 2.5-1.5 (m, 26H)).

### 2. Synthesis of Compound 13

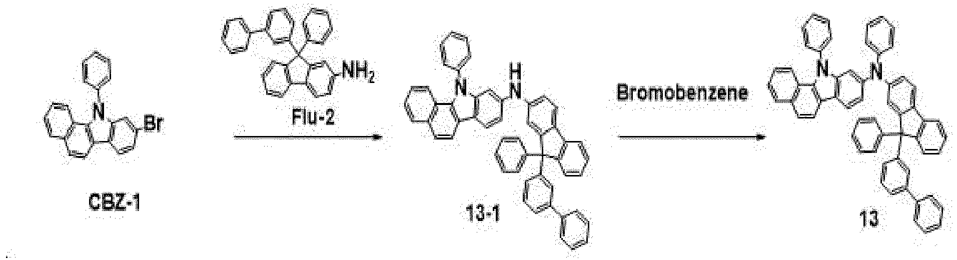

### (1) Synthesis of Intermediate 13-1

Intermediate 13-1 (12.6 g, 18 mmol), with a yield of 90%, was obtained in the same manner as in the synthesis of Intermediate 1-1, except that Flu-2 was utilized instead of Flu-1.

### (2) Synthesis of Compound 13

Compound 13 (5.6 g, 9 mmol), with a yield of 90%, was obtained in the same manner as in the synthesis of Compound 1, except that Intermediate 13-1 was utilized instead of Intermediate 1-1. (C₅₉H₄₀N₂ M+1: 776.32, ¹H NMR (500 MHz, CDCl₃) δ= 7.80 (m, 2H), 7.60 (d, 1H), 7.55-7.10(m, 22H), 2.5-1.5 (m, 26H)).

### 3. Synthesis of Compound 38

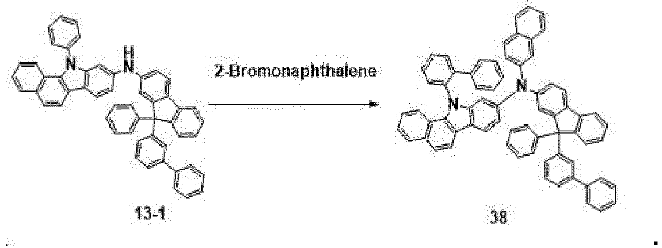

Compound 38 (8.126 g, 9 mmol), with a yield of 90%, was obtained in the same manner as in the synthesis of Compound 13, except that 2-bromonaphthalene was utilized instead of bromobenzene. (C₆₉H₄₆N₂ M+1: 902.37, ¹H NMR (500 MHz, CDCl₃) δ= 7.80 (m, 2H), 7.60 (d, 1H), 7.55-7.10(m, 22H), 2.5-1.5 (m, 26H)).

### 4. Synthesis of Compound 62

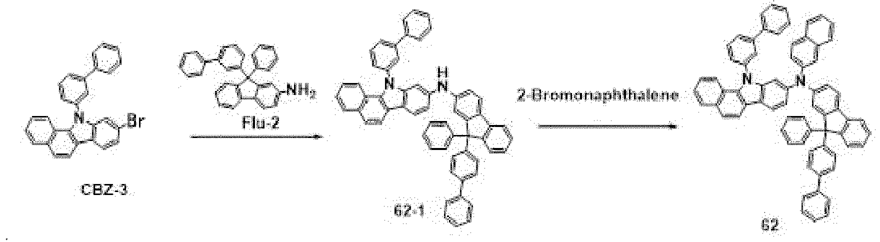

### (1) Synthesis of Intermediate 62-1

Intermediate 62-1 (13.97 g, 18 mmol), with a yield of 90%, was obtained in the same manner as in the synthesis of Intermediate 13-1, except that CBZ-3 was utilized instead of CBZ-1.

### (2) Synthesis of Compound 62

Compound 62 (5.6 g, 9 mmol), with a yield of 90%, was obtained in the same manner as in the synthesis of Compound 38, except that Intermediate 62-1 was utilized instead of Intermediate 13-1. (C₆₉H₄₆N₂ M+1: 902.37, ¹H NMR (500 MHz, CDCl₃) δ= 7.80 (m, 2H), 7.60 (d, 1H), 7.55-7.10(m, 22H), 2.5-1.5 (m, 26H)).

### 5. Synthesis of Compound 86

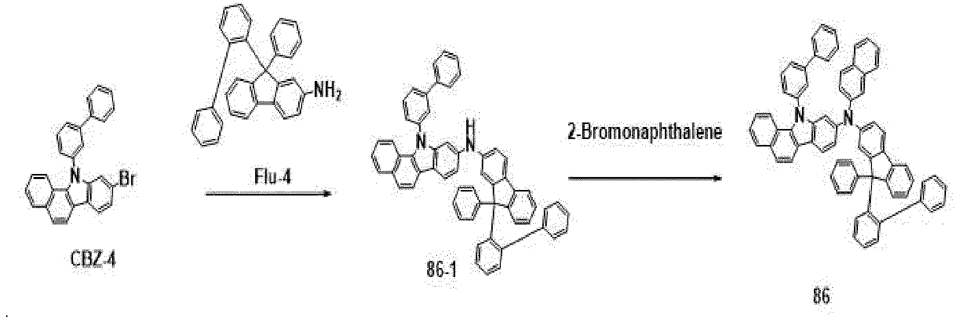

### (1) Synthesis of Intermediate 86-1

Intermediate 86-1 (13.9 g, 18 mmol), with a yield of 90%, was obtained in the same manner as in the synthesis of Intermediate 1-1, except that CBZ-4 was utilized instead of CBZ-1 and Flu-4 was utilized instead of Flu-1.

### (2) Synthesis of Compound 86

Compound 86 (8.12 g, 9 mmol), with a yield of 90%, was obtained in the same manner as in the synthesis of Compound 38, except that Intermediate 86-1 was utilized instead of Intermediate 13-1. (C₆₉H₄₆N₂ M+1: 902.37, ¹H NMR (500 MHz, CDCl₃) δ= 7.80 (m, 2H), 7.60 (d, 1H), 7.55-7.10(m, 22H), 2.5-1.5 (m, 26H)).

### 6. Synthesis of Compound 103

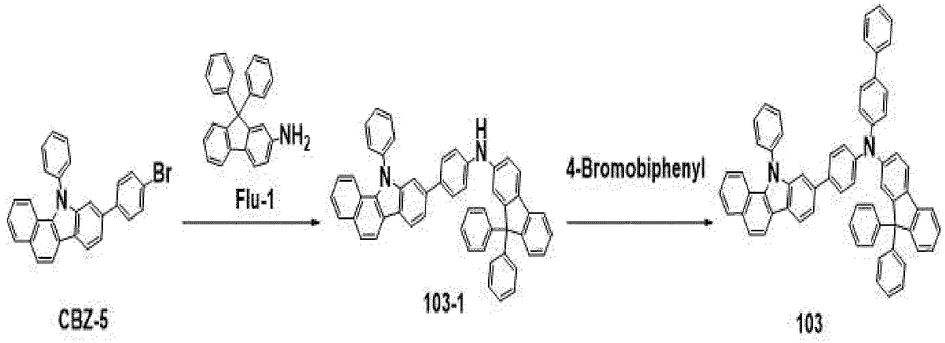

### (1) Synthesis of Intermediate 103-1

Intermediate 103-1 (12.6 g, 18 mmol), with a yield of 90%, was obtained in the same manner as in the synthesis of Intermediate 1-1, except that CBZ-5 was utilized instead of CBZ-1.

### (2) Synthesis of Compound 103

Compound 103 (7.67 g, 9 mmol), with a yield of 90%, was obtained in the same manner as in the synthesis of Compound 1, except that Intermediate 103-1 was utilized instead of Intermediate 1-1 and 4-bromobiphenyl was utilized instead of bromobenzene. (C₆₅H₄₄N₂ M+1: 852.32, ¹H NMR (500 MHz, CDCl₃) δ= 7.80 (m, 2H), 7.60 (d, 1H), 7.55-7.10(m, 22H), 2.5-1.5 (m, 26H)).

### 7. Synthesis of Compound 116

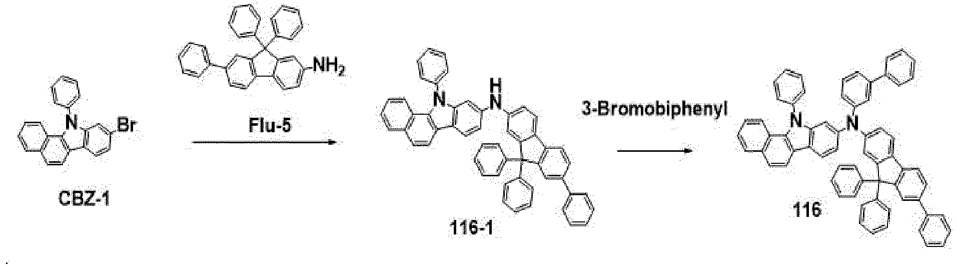

### (1) Synthesis of Intermediate 116-1

Intermediate 116-1 (12.6 g, 18 mmol), with a yield of 90%, was obtained in the same manner as in the synthesis of Intermediate 1-1, except that Flu-5 was utilized instead of Flu-1.

### (2) Synthesis of Compound 116

Compound 116 (7.67 g, 9 mmol), with a yield of 90%, was obtained in the same manner as in the synthesis of Compound 1, except that Intermediate 116-1 was utilized instead of Intermediate 1-1 and 3-bromobiphenyl was utilized instead of bromobenzene. (C₆₅H₄₄N₂ M+1: 852.32, ¹H NMR (500 MHz, CDCl₃) δ= 7.80 (m, 2H), 7.60 (d, 1H), 7.55-7.10(m, 22H), 2.5-1.5 (m, 26H)).

### Device Manufacturing Example

An organic electroluminescence device was manufactured utilizing compounds of Examples and Comparative Examples below as a material for a hole transport region.

### Example Compounds

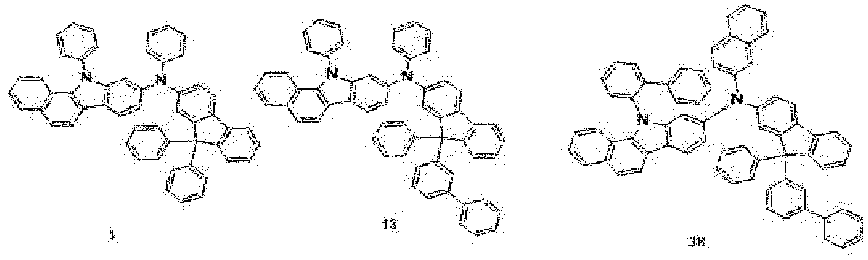

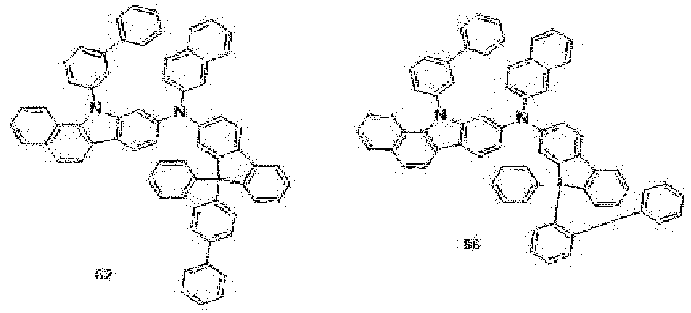

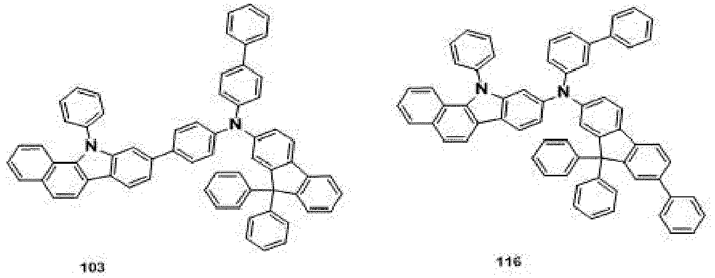

### Comparative Example Compounds

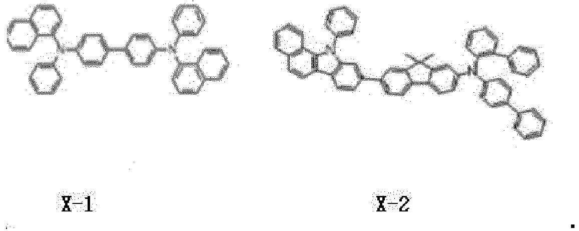

Organic electroluminescence devices of Examples and Comparative Examples were manufactured through the following method. As an anode, an ITO glass substrate (corning, 15 Ω/cm², 1200 Å) was cut to a size of about 50 mm x 50 mm × 0.7 mm, subjected to ultrasonic cleaning utilizing isopropyl alcohol and pure water for 5 minutes respectively and ultraviolet irradiation for 30 minutes, and then exposed to ozone for cleaning to form the glass substrate in a vacuum deposition apparatus.

2-TNATA was vacuum deposited on an upper portion of the substrate at a thickness of 600 Å, and then compounds of Examples or Comparative Examples were vacuum deposited at a thickness of 300 Å to form a hole transport layer.

9,10-di(naphthalen-2-yl)anthracene (hereinafter, ADN) as a suitable (e.g., known) blue fluorescent host and a suitable (e.g., known)compound, 4,4'-bis[2-(4-(N,N-diphenylamino)phenyl)vinyl]biphenyl (hereinafter, DPAVBi) as a blue fluorescent dopant were co-deposited at a weight ratio of 98:2 on the upper portion of the hole transport layer to form an emission layer having a thickness of 300 Å.

Then, Alq₃ was deposited on the emission layer to form an electron transport layer having a thickness of 300 Å, and then LiF, which is a halogenated alkali metal, was deposited to form an electron injection layer having a thickness of 10 Å, and Al was vacuum deposited at a thickness of 3000 Å, thereby forming a second electrode (cathode electrode) of LiF/Al. Each layer was formed through vacuum deposition.

The measured values according to Examples 1 to 7 and Comparative Examples 1 and 2 are shown in Table 1 below. Luminous efficiency was measured at 10mA/cm², and half life was expressed as a luminance half-life from an initial luminance of 1000 cd/cm². The measured values according to Examples 1 to 7 and Comparative Examples 1 and 2 are shown in Table 1 below.

**Table 1**

| | Hole transpor t material | Driving voltage (V) | Current density (mA/cm) | Luminanc e (cd/m²) | Efficiency (cd/A) | Emitte d color | Half life (hr @100 mA/ cm) |
|---|---|---|---|---|---|---|---|
| Comparati ve Example 1 | X-1 | 7.01 | 50 | 2645 | 5.29 | blue | 258 |
| Comparati ve Example 2 | X-2 | 5.62 | 50 | 3225 | 6.45 | blue | 280 |
| Example 1 | Compou nd 1 | 4.32 | 50 | 3670 | 7.34 | blue | 362 |
| Example 2 | Compou nd 13 | 4.21 | 50 | 3715 | 7.43 | blue | 353 |
| Example 3 | Compou nd 38 | 4.22 | 50 | 3665 | 7.33 | blue | 372 |
| Example 4 | Compou nd 62 | 4.26 | 50 | 3730 | 7.46 | blue | 374 |
| Example 5 | Compou nd 86 | 4.26 | 50 | 3730 | 7.46 | blue | 374 |
| Example 6 | Compou nd 103 | 4.25 | 50 | 3630 | 7.26 | blue | 384 |
| Example 7 | Compou nd 116 | 4.41 | 50 | 3725 | 7.45 | blue | 343 |

Referring to Table 1, it is seen that all of Examples 1 to 7 achieve lower driving voltage, longer half life, higher luminance and higher efficiency as compared to Comparative Examples 1 and 2.

The nitrogen-containing compound according to an embodiment of the present disclosure is utilized in the hole transport region to accomplish low voltage, high efficiency, and long lifespan of a luminescence device. For example, the lifespan of the luminescence device including the nitrogen-containing compound according to an embodiment of the present disclosure in the hole transport region is remarkably increased.

The nitrogen-containing compound according to an embodiment of the present disclosure is a compound containing condensed rings in the molecular structure, and thus has a relatively high glass transition temperature (Tg) or melting point due to the introduction of the condensed rings. Accordingly, the nitrogen-containing compound according to an embodiment of the present disclosure has excellent heat resistance in a high-temperature setting generated at the interlayer interface when a luminescence device is in operation (e.g., emitting light), and may thus improve durability of the luminescence device. When the nitrogen-containing compound according to an embodiment of the present disclosure is utilized as a hole transport layer adjacent to an emission layer of a blue phosphorescent luminescence device specifically, high efficiency and long lifespan may be achieved.

A luminescence device according to an embodiment of the present disclosure has excellent efficiency.

A nitrogen-containing compound according to an embodiment of the present disclosure may be utilized as a material for a hole transport region of a luminescence device, and when the nitrogen-containing compound is utilized, the luminescence device may have increased efficiency.

The use of "may" when describing embodiments of the inventive concept refers to "one or more embodiments of the inventive concept."

As used herein, the term "substantially," "about," and similar terms are used as terms of approximation and not as terms of degree, and are intended to account for the inherent deviations in measured or calculated values that would be recognized by those of ordinary skill in the art. "About" or "approximately," as used herein, is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, 5% of the stated value.

Also, any numerical range recited herein is intended to include all subranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein.

The apparatus and/or any other relevant devices or components according to embodiments of the present invention described herein may be implemented utilizing any suitable hardware, firmware (e.g. an application-specific integrated circuit), software, or a combination of software, firmware, and hardware. For example, the various components of the apparatus may be formed on one integrated circuit (IC) chip or on separate IC chips. Further, the various components of the apparatus may be implemented on a flexible printed circuit film, a tape carrier package (TCP), a printed circuit board (PCB), or formed on one substrate. Further, the various components of the apparatus may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random access memory (RAM). The computer program instructions may also be stored in other non-transitory computer readable media such as, for example, a CD-ROM, flash drive, or the like. Also, a person of skill in the art should recognize that the functionality of various computing devices may be combined or integrated into a single computing device, or the functionality of a particular computing device may be distributed across one or more other computing devices without departing from the scope of the embodiments.

Although the embodiments of the present disclosure have been described above, those skilled in the art to which the present disclosure pertains may implement the present disclosure in other specific forms without changing the technical idea or essential features thereof. Therefore, it should be understood that the embodiments described above are not restrictive.

Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope as defined by the following claims, and equivalents thereof.

## Claims

1. A nitrogen-containing compound represented by Formula 1: 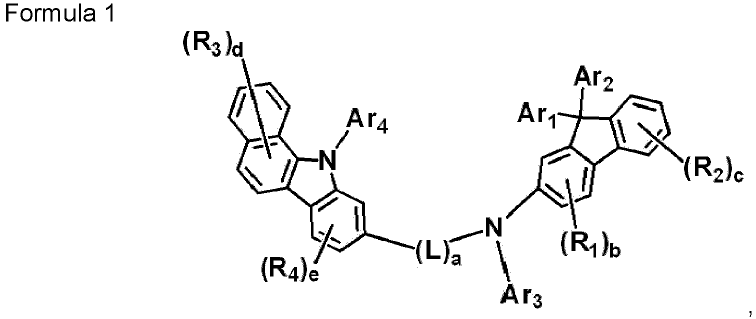 and wherein in Formula 1,
L is a direct linkage, a substituted or unsubstituted divalent hydrocarbon ring group having 6 to 30 ring-forming carbon atoms, a substituted or unsubstituted arylene group having 6 to 30 ring-forming carbon atoms, a substituted or unsubstituted divalent aliphatic heterocyclic group having 2 to 30 ring-forming carbon atoms, or a substituted or unsubstituted heteroarylene group having 2 to 30 ring-forming carbon atoms,
Ar₁ to Ar₄ are each independently a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms,
R₁ to R₄ are each independently a hydrogen atom, a deuterium atom, a halogen atom, a nitro group, a cyano group, an oxy group, a substituted or unsubstituted amine group, a substituted or unsubstituted thio group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms,
a, b, and e are each independently an integer of 0 to 3,
c is an integer of 0 to 4, and
d is an integer of 0 to 6.

2. The nitrogen-containing compound of claim 1, wherein Ar₁ to Ar₄ are each independently a substituted or unsubstituted aryl group having 6 to 30 ring-forming carbon atoms.

3. The nitrogen-containing compound of claim 1 or claim 2, wherein L in Formula 1 is a direct linkage or a substituted or unsubstituted arylene group having 6 to 18 ring-forming carbon atoms.

4. The nitrogen-containing compound of claim 1, wherein the nitrogen-containing compound represented by Formula 1 is represented by Formula 2-1 or Formula 2-2: 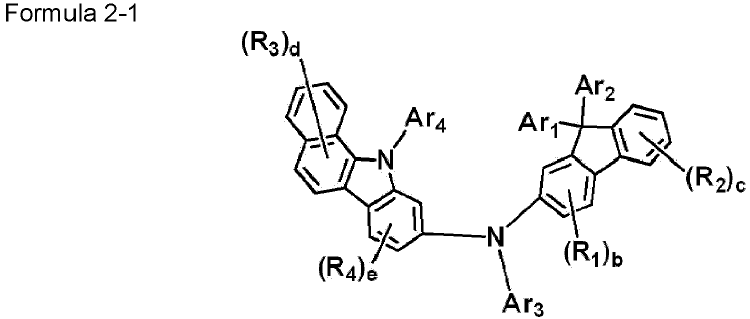 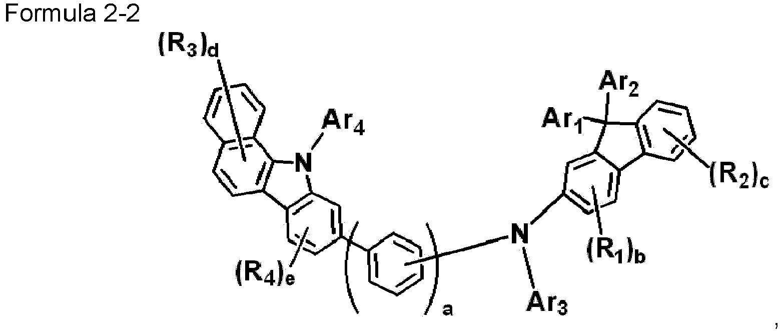 and wherein in Formulae 2-1 and 2-2 above,
Ar₁ to Ar₄, R₁ to R₄, and a to e are the same as respectively defined in Formula 1.

5. The nitrogen-containing compound of claim 1, wherein the nitrogen-containing compound represented by Formula 1 is represented by Formula 3: 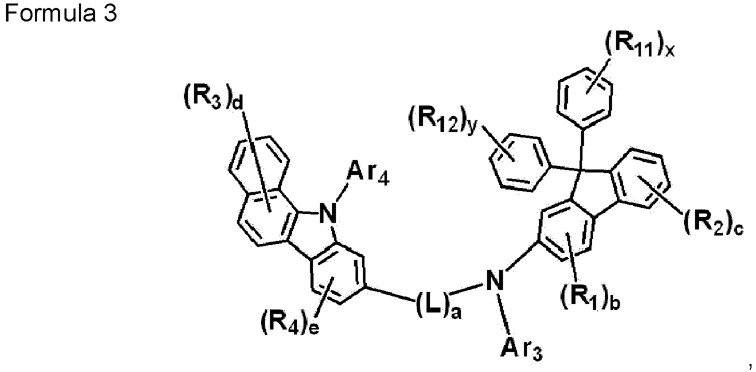 and wherein in Formula 3,
R₁₁ and R₁₂ are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring,
x and y are each independently an integer of 0 to 5, and
Ar₃, Ar₄, R₁ to R₄, L, and a to e are the same as respectively defined in Formula 1.

6. The nitrogen-containing compound of any one of claims 1 to 5, wherein Ar₃ and Ar₄ are each independently represented by any one of Formulae 4-1 to 4-4: Formulae 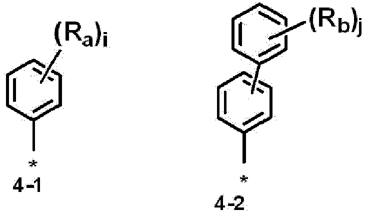 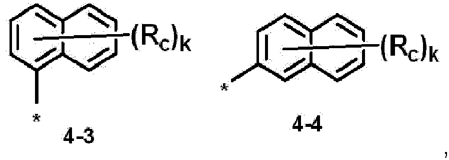 and wherein in Formulae 4-1 to 4-4,
Rₐ to R_{c} are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring,
i and j are each independently an integer of 0 to 5, and
k is an integer of 0 to 7.

7. The nitrogen-containing compound of claim 5, wherein the nitrogen-containing compound represented by Formula 3 is represented by Formula 5: Formula 5 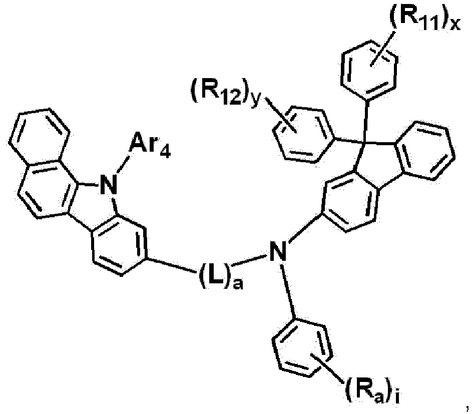 and wherein in Formula 5,
Rₐ is a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; and/or bonded to an adjacent group to form a ring,
i is an integer of 0 to 5, and
Ar₄, R₁₁, R₁₂, L, a, x, and y are the same as respectively defined in Formula 3.

8. The nitrogen-containing compound of claim 1, wherein the nitrogen-containing compound represented by Formula 1 is at least one selected from compounds represented by Compound Group 1:

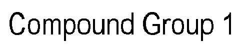

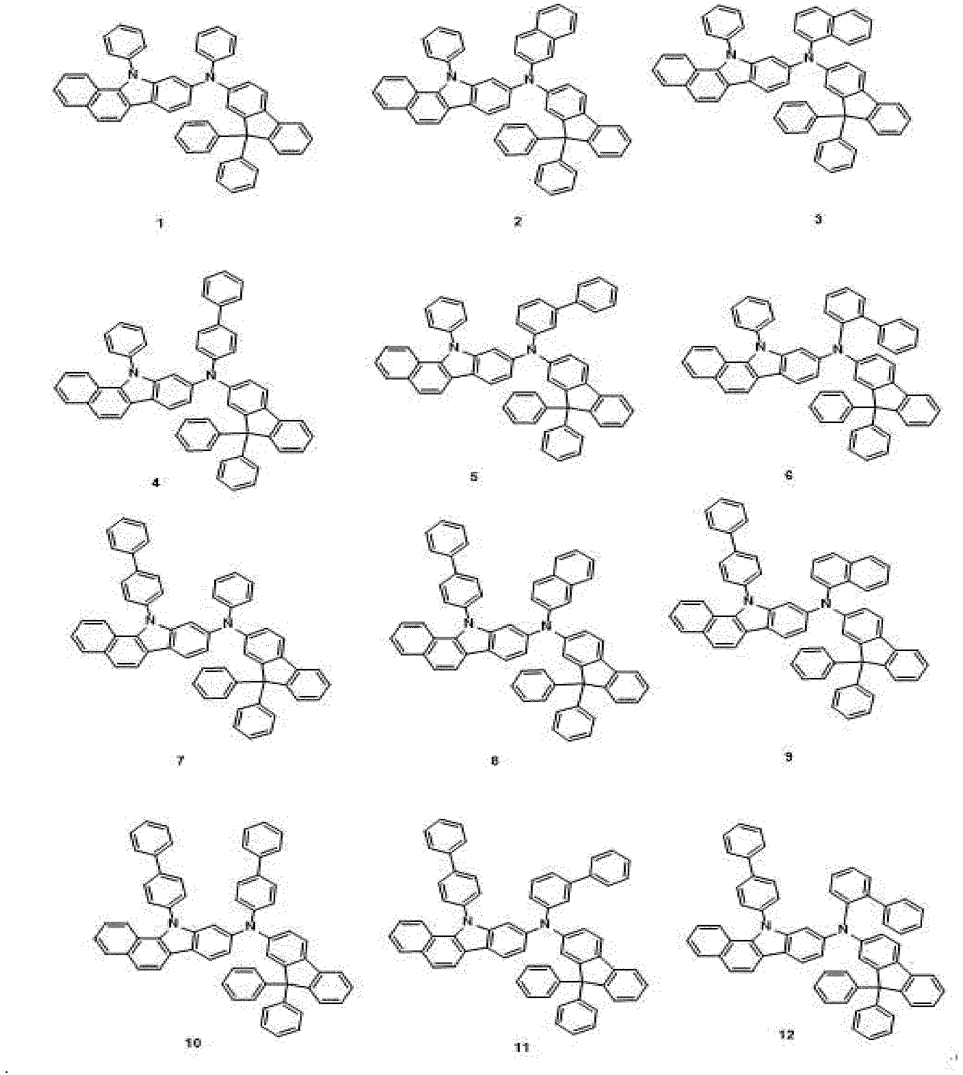

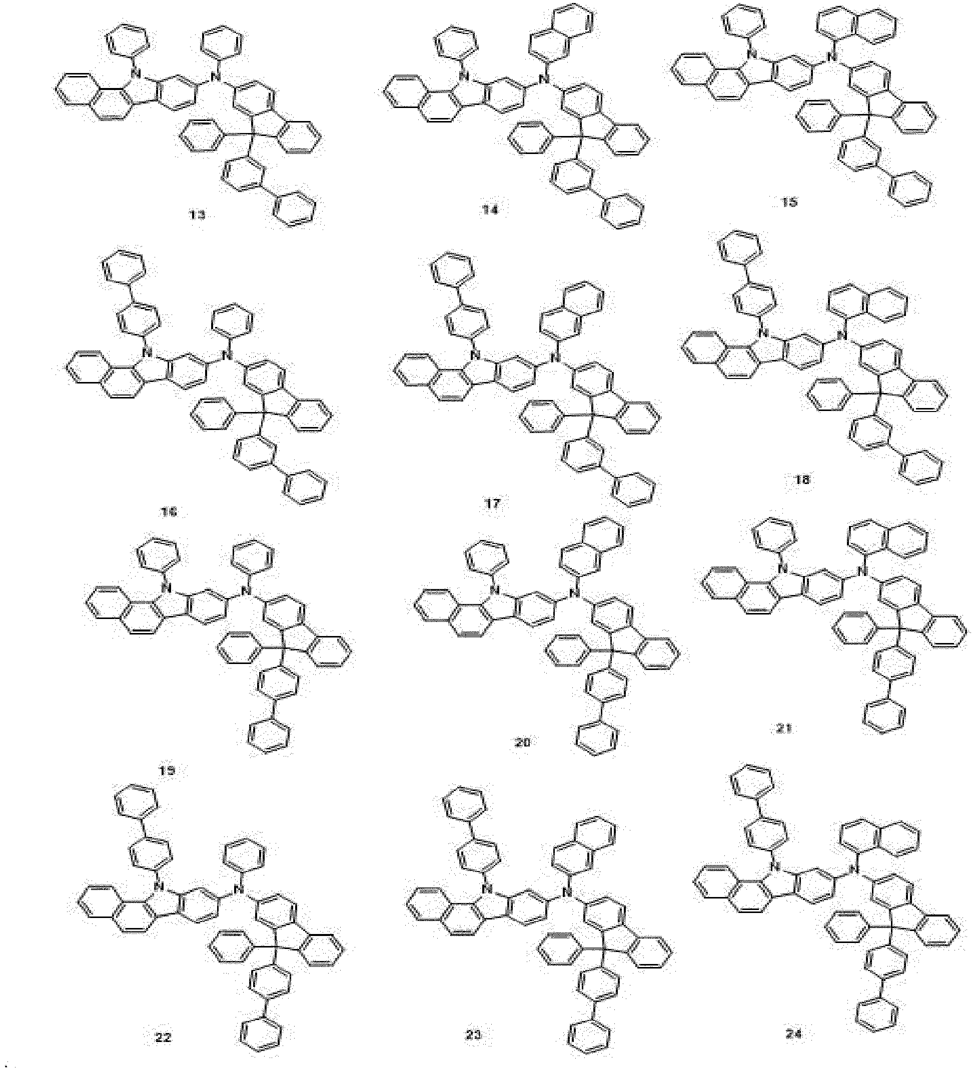

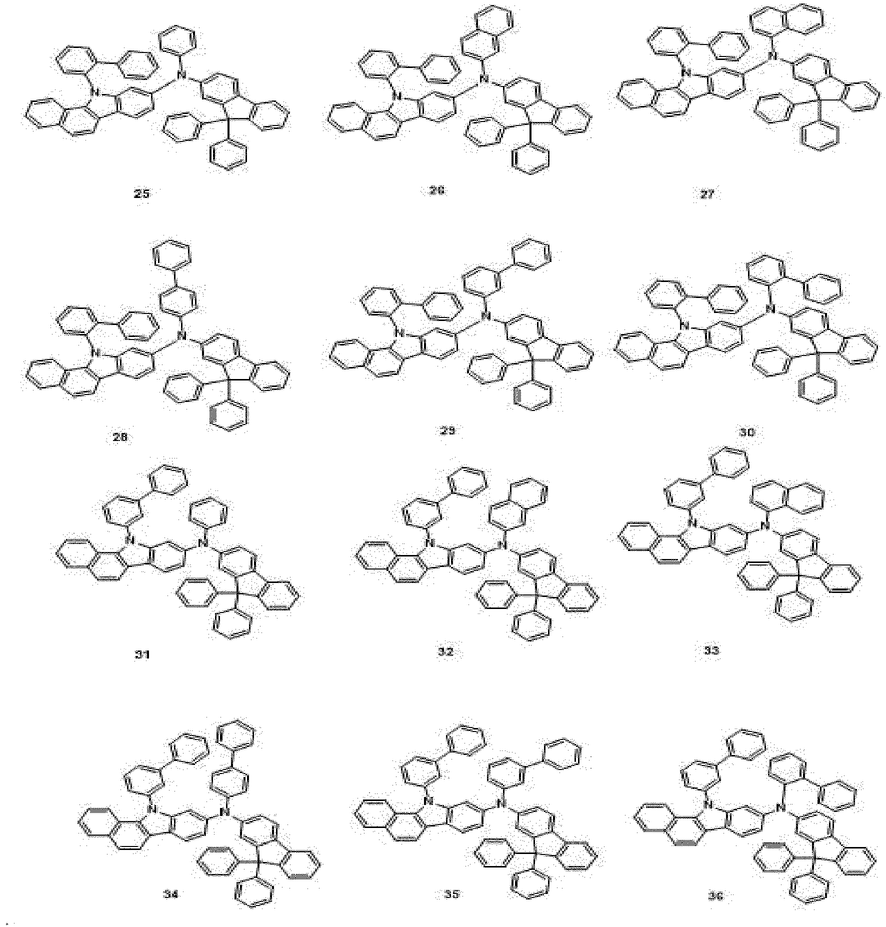

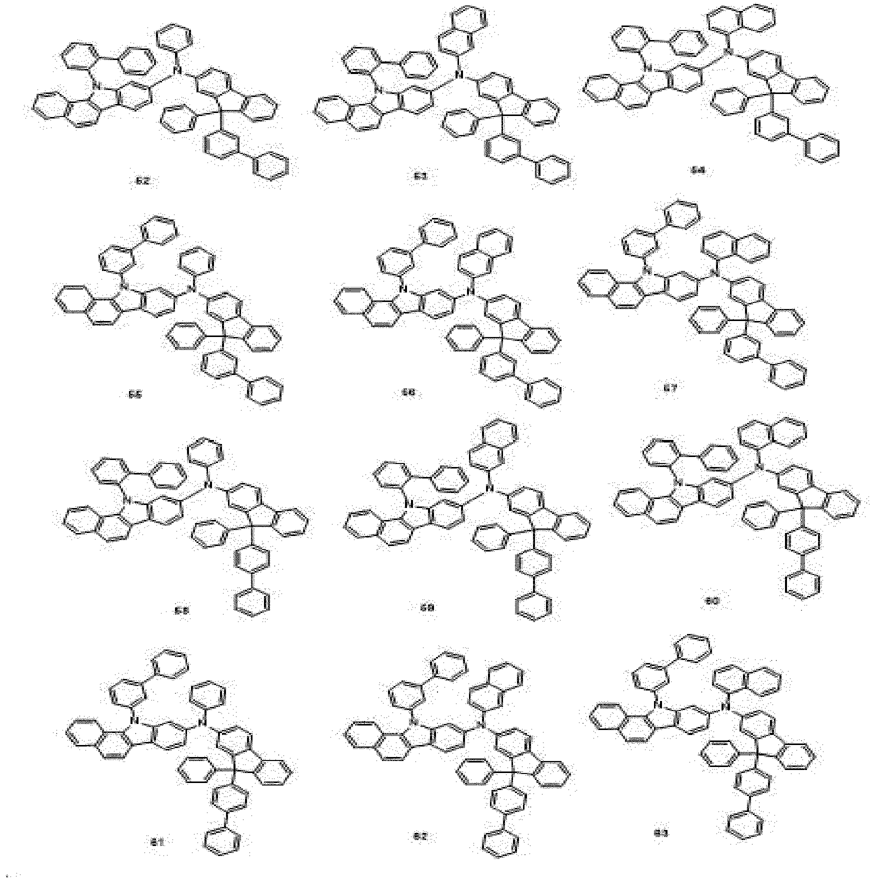

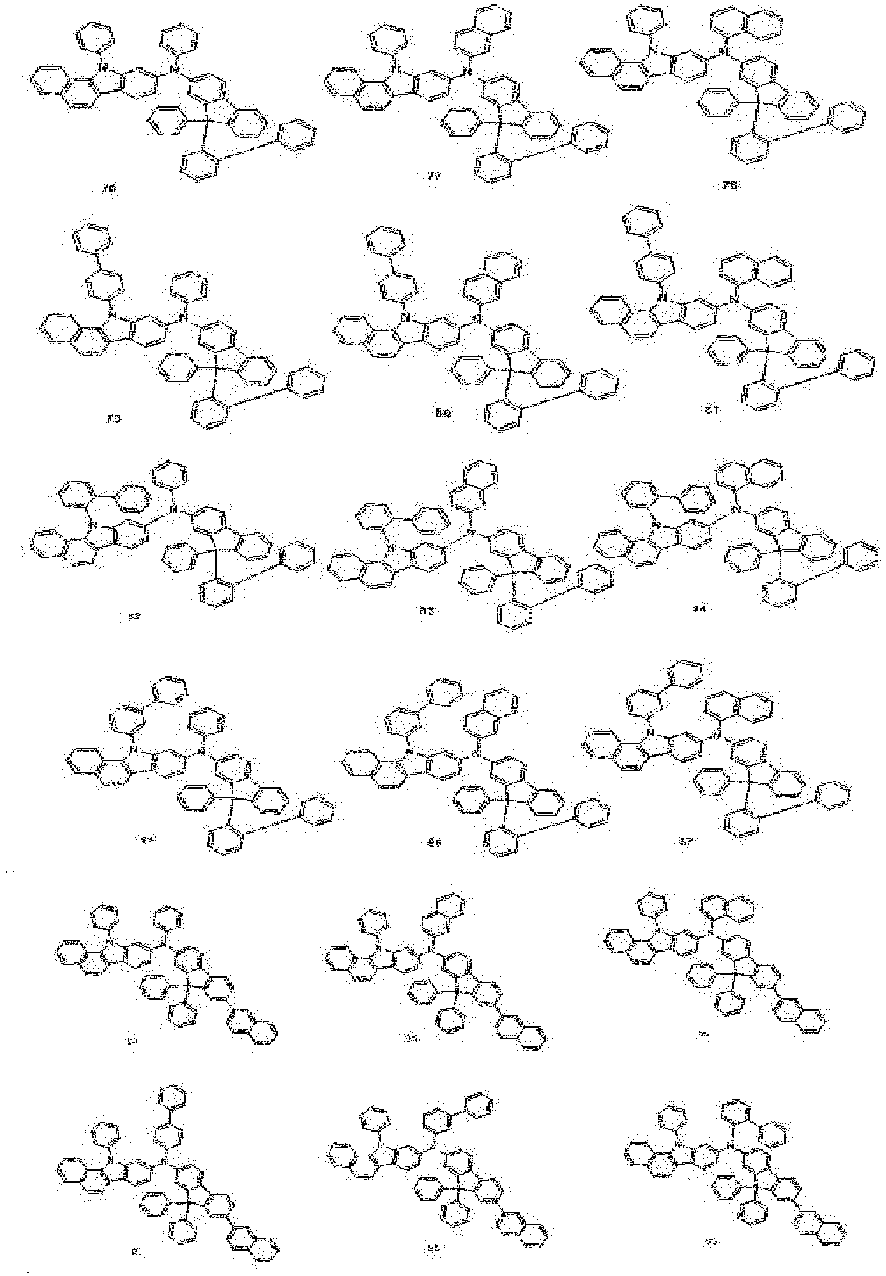

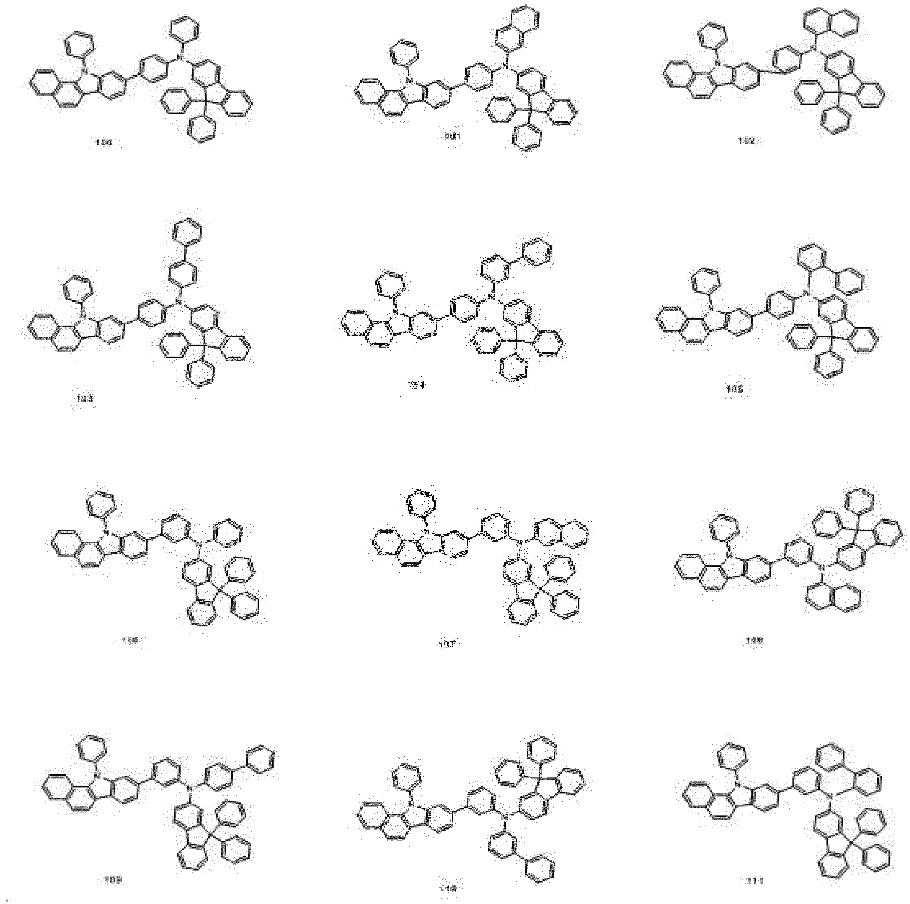

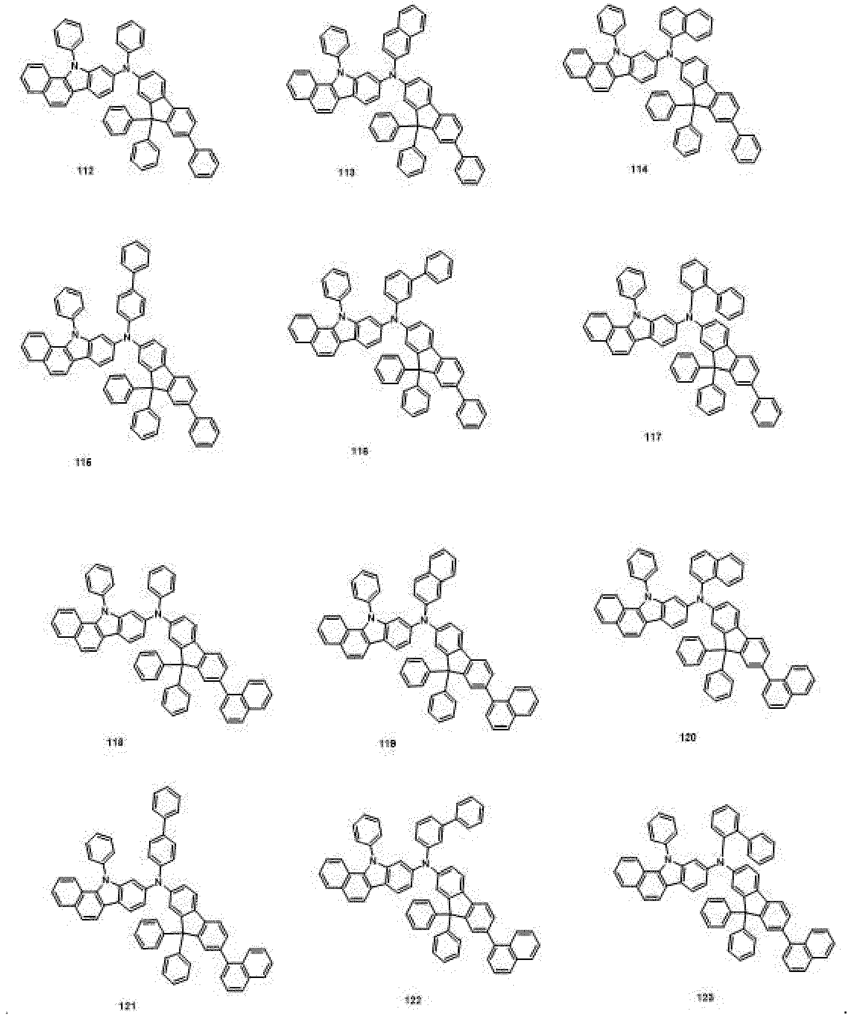

9. A luminescence device comprising:
a first electrode;
a hole transport region on the first electrode;
an emission layer on the hole transport region;
an electron transport region on the emission layer; and
a second electrode on the electron transport region,
wherein the hole transport region comprises a nitrogen-containing compound according to any one of claims 1 to 8.

10. The luminescence device of claim 9, wherein the hole transport region comprises:
a hole injection layer on the first electrode; and
a hole transport layer on the hole injection layer,
the hole injection layer and/or the hole transport layer comprising the nitrogen-containing compound.
